(19) Europäisches Patentamt
European Patent Office
Office européen des brevets

(11) EP 1 333 025 A1

(12) EUROPEAN PATENT APPLICATION
published in accordance with Art. 158(3) EPC

(43) Date of publication:
**06.08.2003 Bulletin 2003/32**

(21) Application number: **01981047.2**

(22) Date of filing: **09.11.2001**

(51) Int Cl.7: **C07D 207/16**, C07D 401/12, C07D 401/06, A61K 31/401, A61K 31/4439, A61K 31/4725, A61P 43/00, A61P 3/10, A61P 37/02, A61P 29/00, A61P 31/18, A61P 35/04

(86) International application number:
**PCT/JP01/09818**

(87) International publication number:
**WO 02/038541 (16.05.2002 Gazette 2002/20)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **10.11.2000 JP 2000344036**
**16.07.2001 JP 2001215766**

(71) Applicant: **TAISHO PHARMACEUTICAL CO., LTD**
**Tokyo 170-8633 (JP)**

(72) Inventors:
- **FUKUSHIMA, Hiroshi,**
  **Taisho Pharmaceutical Co. Ltd.**
  **Tokyo 170-8633 (JP)**
- **HIRATATE, Akira,**
  **Taisho Pharmaceutical Co. Ltd.**
  **Tokyo 170-8633 (JP)**
- **TAKAHASHI, Masato,**
  **Taisho Pharmaceutical Co. Ltd.**
  **Tokyo 170-8633 (JP)**
- **KAMEO, Kazuya,**
  **c/o Taisho Pharmaceutical Co. Ltd.**
  **Tokyo 170-8633 (JP)**

(74) Representative: **HOFFMANN - EITLE**
**Patent- und Rechtsanwälte**
**Arabellastrasse 4**
**81925 München (DE)**

# (54) CYANOPYRROLIDINE DERIVATIVES

(57) A cyanopyrrolidine derivative represented by Formula (1):

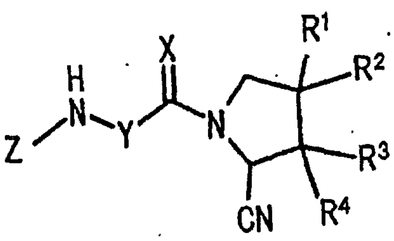

(1)

[wherein $R^1$ is a halogen atom, a hydroxyl group, an alkoxy group having 1 to 5 carbon atoms or an alkyl group having 1 to 5 carbon atoms, $R^2$ is a hydrogen atom, a halogen atom, a hydroxyl group, an alkoxy group having 1 to 5 carbon atoms or an alkyl group having 1 to 5 carbon atoms, or $R^1$ and $R^2$ together form an oxo, a hydroxyimino group, an alkoxyimino group having 1 to 5 carbon atoms or an alkylidene group having 1 to 5 carbon atoms,

$R^3$ and $R^4$ are each a hydrogen atom, a halogen atom, a hydroxyl group, an alkoxy group having 1 to 5 carbon atoms or an alkyl group having 1 to 5 carbon atoms, or $R^3$ and $R^4$ together form an oxo, a hydroxyimino group, an alkoxyimino group having 1 to 5 carbon atoms or an alkylidene group having 1 to 5 carbon atoms,

X is an oxygen atom or a sulfur atom,

EP 1 333 025 A1

Y is $-CR^5R^6-$ (wherein $R^5$ and $R^6$ are the same or different, and are each a hydrogen atom, a halogen atom, an optionally substituted alkyl group having 1 to 10 carbon atoms or an optionally substituted alkenyl group having 2 to 10 carbon atoms), or $-CR^7R^8-CR^9R^{10}-$ (wherein $R^7$, $R^8$, $R^9$ and $R^{10}$ are the same or different, and each a hydrogen atom, a halogen atom or an optionally substituted alkyl group having 1 to 10 carbon atoms, or $R^7$ and $R^9$ together with the carbon atom to which they are attached form an optionally substituted cycloalkyl group having 3 to 8 carbon atoms, an optionally substituted cycloalkenyl group having 4 to 8 carbon atoms, an optionally substituted bicycloalkyl group having 5 to 10 carbon atoms, or an optionally substituted bicycloalkenyl group having 5 to 10 carbon atoms) and

Z is a hydrogen atom or an optionally substituted alkyl group having 1 to 10 carbon atoms, or Y and Z together with the nitrogen atom to which they are attached form an optionally substituted cyclic amino group having 2 to 10 carbon atoms], or a pharmaceutically acceptable salt thereof.

## Description

TECHNICAL FIELD

The present invention relates to novel cyanopyrrolidine derivatives.

BACKGROUND ART

Dipeptidyl peptidase IV (DPP IV) is a kind of serine proteases which can hydrolyze the dipeptide from the peptide chain having proline or alanine at the second position from the N-terminus. DPP IV is distributed in a variety of the tissues (including kidney or liver) and plasma, and participates in the metabolisms of various physiologically active peptides.

Recently, it has been made clear that DPP IV acts on the metabolism of glucagon-like peptide-1 (GLP-1). That is, DPP IV hydrolyzes the dipeptide of the N-terminal His-Ala of GLP-1, thereby, GLP-1 is inactivated, and the inactivated product acts as an antagonist of GLP-1 receptor.

GLP-1 has been found to have physiological actions such as an accelerating action of insulin-secretion from the pancreas, a prolonging action of gastric emptying time or an inhibitory action of eating. Accordingly, DPP IV inhibition elevates the GLP-1 effect, enhances the insulin effect and improves glucose metabolism, therefore, DPP IV inhibition is expected to be useful for treating type 2 diabetes mellitus.

In addition, DPP IV has been found to participate in the metabolism of neuropeptide Y which is a kind of neuropeptides, activation of T cells which are immunocompetent cells, adhesion of cancer cells to the endothelium or invasion of HIV virus into lymphocytes. Accordingly, DPP IV inhibition is considered to be useful for treating immune diseases, etc.

Furthermore, a high level of DPP IV expression has been found in fibroblasts of the skin of human subjects of psoriasis, rheumatoid arthritis and lichen planus, and a high DPP IV activity has been found in the subjects of benign prostatic hypertrophy. Accordingly, DPP IV inhibition is also expected to be effective to skin diseases and benign prostatic hypertrophy.

Among DPP IV inhibiting compounds which have been known up to this time, there are the compounds which are substituted with a phosphorus atom at the 2-position of pyrrolidine (J. Med. Chem., 37, 3969 - 3976, 1994), and the compounds which are substituted with a boron atom at the 2-position of pyrrolidine (Biochemistry, 32, 8723 - 8731, 1993). Also are known the compounds which are substituted with a cyano group at the 2-position of pyrrolidine (Arch. Biochem. Biophys., 323, 148 - 152, 1995; Bioorg. Med. Chem. Lett., 6, 1163 - 1166, 1996; Biochemistry, 38, 11597 - 11603, 1999), but there is no report on any inhibitors which have substituent(s) at the 3- or 4-position of 2-cyanopyrrolidine derivatives.

An object of the present invention is to provide novel cyanopyrrolidine derivatives which have an excellent DPP IV inhibition activity.

DISCLOSURE OF THE INVENTION

As a result of the continued extensive studies in order to achieve the above-mentioned object, the present inventors have found that certain cyanopyrrolidine derivatives have an excellent DPP IV inhibition activity, and thereby the present invention has been accomplished.

One aspect of the present invention is to provide a compound represented by Formula (1) (hereinafter referred to as "the compound of the present invention" or "the present invention compound"):

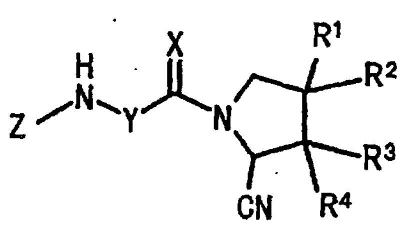

(1)

[wherein $R^1$ is a halogen atom, a hydroxyl group, an alkoxy group having 1 to 5 carbon atoms or an alkyl group having 1 to 5 carbon atoms, $R^2$ is a hydrogen atom, a halogen atom, a hydroxyl group, an alkoxy group having 1 to 5 carbon

atoms or an alkyl group having 1 to 5 carbon atoms, or $R^1$ and $R^2$ together form an oxo, a hydroxyimino group, an alkoxyimino group having 1 to 5 carbon atoms or an alkylidene group having 1 to 5 carbon atoms,

$R^3$ and $R^4$ are each a hydrogen atom, a halogen atom, a hydroxyl group, an alkoxy group having 1 to 5 carbon atoms or an alkyl group having 1 to 5 carbon atoms, or $R^3$ and $R^4$ together form an oxo, a hydroxyimino group, an alkoxyimino group having 1 to 5 carbon atoms or an alkylidene group having 1 to 5 carbon atoms,

X is an oxygen atom or a sulfur atom,

Y is $-CR^5R^6-$ [wherein $R^5$ and $R^6$ are the same or different, and each a hydrogen atom; a halogen atom; an alkyl group having 1 to 10 carbon atoms which is optionally substituted with at least one selected from the group consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), $-CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a chain alkyl group having 1 to 5 carbon atoms or a benzyl group); or an alkenyl group having 2 to 10 carbon atoms which is optionally substituted with at least one selected from the group consisting of a halogen atom, a hydroxyl group, a carboxyl group, an amino group, an aminocarbonyl group and a chain alkoxy group having 1 to 5 carbon atoms], or $-CR^7R^8-CR^9R^{10}-$ (wherein $R^7$, $R^8$, $R^9$ and $R^{10}$ are the same or different, and each a hydrogen atom; a halogen atom; or an alkyl group having 1 to 10 carbon atoms which is optionally substituted with at least one selected from the group consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), $-CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a chain alkyl group having 1 to 5 carbon atoms or a benzyl group), or $R^7$ and $R^9$ together with the carbon atom to which they are attached form a cycloalkyl group having 3 to 8 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms; a cycloalkenyl group having 4 to 8 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms; a bicycloalkyl group having 5 to 10 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms; or a bicycloalkenyl group having 5 to 10 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms), and

Z is a hydrogen atom; or an alkyl group having 1 to 10 carbon atoms which is optionally substituted with at least one selected from the group consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), $-CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a chain alkyl group having 1 to 5 carbon atoms or a benzyl group), or Y and Z together with the nitrogen atom to which they are attached form a cyclic amino group having 2 to 10 carbon atoms which is optionally substituted with at least one selected from the group consisting of a halogen atom, a hydroxyl group, an amino group, a chain alkyl group having 1 to 5 carbon atoms and $-OR^{15}$ (wherein $R^{15}$ is a chain alkyl group having 1 to 5 carbon atoms, an aminocarbonylmethyl group or a benzyl group)] or a pharmaceutically acceptable salt thereof.

## BEST MODE FOR CARRYING OUT THE INVENTION

Another aspect of the present invention is to provide the cyanopyrrolidine derivative of Formula (1) wherein $R^1$ is a halogen atom, a hydroxyl group, an alkoxy group having 1 to 5 carbon atoms or an alkyl group having 1 to 5 carbon atoms, and $R^2$, $R^3$ and $R^4$ are each a hydrogen atom, a halogen atom, a hydroxyl group, an alkoxy group having 1 to 5 carbon atoms or an alkyl group having 1 to 5 carbon atoms, or the pharmaceutically acceptable salt thereof.

A still another aspect of the present invention is to provide the cyanopyrrolidine derivative of Formula (1)

wherein $R^1$ is a fluorine atom or a chlorine atom, or the pharmaceutically acceptable salt thereof.

A further aspect of the present invention is to provide the cyanopyrrolidine derivative of Formula (1) wherein $R^1$ is a fluorine atom, and $R^2$ is a hydrogen atom, or the pharmaceutically acceptable salt thereof.

A still further aspect of the present invention is to provide the cyanopyrrolidine derivative of Formula (1) wherein $R^1$ is a fluorine atom, and $R^2$, $R^3$ and $R^4$ are each a hydrogen atom, or the pharmaceutically acceptable salt thereof.

A still further aspect of the present invention is to provide a cyanopyrrolidine derivative represented by Formula (2):

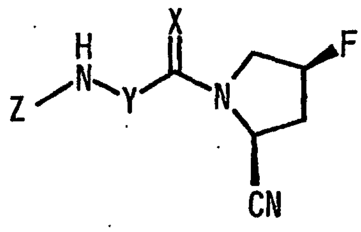

(2)

wherein X, Y and Z are defined as above) or a pharmaceutically acceptable salt thereof.

A still further aspect of the present invention is to provide the cyanopyrrolidine derivative of Formula (1) or (2) wherein X is an oxygen atom, or the pharmaceutically acceptable salt thereof.

A still further aspect of the present invention is to provide the cyanopyrrolidine derivative of Formula (1) or (2) wherein Y is $-CH_2-$, or the pharmaceutically acceptable salt thereof.

A still further aspect of the present invention is to provide the cyanopyrrolidine derivative of Formula (1) or (2) wherein Y is $-CH_2-$ and Z is a branched or cyclic alkyl group having 4 to 10 carbon atoms which is optionally substituted with at least one selected from the group consisting of a hydroxyl group and a hydroxyalkyl group having 1 to 5 carbon atoms, or the pharmaceutically acceptable salt thereof.

A still further aspect of the present invention is to provide the cyanopyrrolidine derivative of Formula (1) or (2) wherein Y is $-CH_2-$ and Z is a tert-butyl group, a (1-hydroxymethyl)cyclopentyl group or a (2-hydroxy-1,1-dimethyl) ethyl group, or the pharmaceutically acceptable salt thereof.

A still further aspect of the present invention is to provide the cyanopyrrolidine derivative of Formula (1) or (2) wherein Y is $-CR^5R^6-$ (wherein $R^5$ is a hydrogen atom) and Z is a hydrogen atom, or the pharmaceutically acceptable salt thereof.

A still further aspect of the present invention is to provide the cyanopyrrolidine derivative of Formula (1) or (2) wherein Y is $-CR^5R^6-$ (wherein $R^5$ is a hydrogen atom, $R^6$ is a branched or cyclic alkyl group having 3 to 6 carbon atoms) and Z is a hydrogen atom, or the pharmaceutically acceptable salt thereof.

A still further of the present invention is to provide the cyanopyrrolidine derivative of Formula (1) or (2) wherein Y is $-CH[CH(CH_3)_2]-$, $-CH[C(CH_3)_3]-$ or $-CH[CH(CH_3)CH_2CH_3]-$ and Z is a hydrogen atom, or the pharmaceutically acceptable salt thereof.

A still further aspect of the present invention is to provide a pharmaceutical preparation which comprises as an effective ingredient the above-mentioned cyanopyrrolidine derivative or the pharmaceutically acceptable salt thereof.

A still further aspect of the present invention is to provide the above-mentioned pharmaceutical preparation for preventing or treating a disease or condition capable of being improved by inhibition of dipeptidyl peptidase IV.

A still further aspect of the present invention is to provide the above-mentioned pharmaceutical preparation wherein the disease or condition capable of being improved by inhibition of dipeptidyl peptidase IV is diabetes mellitus.

A still further aspect of the present invention is to provide the above-mentioned pharmaceutical preparation wherein the disease or condition capable of being improved by inhibition of dipeptidyl peptidase IV is an immune disease.

In the present invention, "chain" means a straight or branched chain.

The halogen atom means a fluorine atom, a chlorine atom, a bromine atom or an iodine atom.

The alkoxy group having 1 to 5 carbon atoms means a straight, branched or cyclic alkoxy group, examples thereof are a methoxy group, an ethoxy group, a propoxy group, an isopropoxy group, a butoxy group, an isobutoxy group, a tert-butoxy group, a cyclopropylmethoxy group, a pentyloxy group and an isopentyloxy group.

The alkyl group having 1 to 5 carbon atoms means a straight, branched or cyclic alkyl group, and examples thereof are a methyl group, an ethyl group, a propyl group, an isopropyl group, a cyclopropyl group, a butyl group, an isobutyl group, a sec-butyl group, a tert-butyl group, a cyclobutyl group, a cyclopropylmethyl group, a pentyl group, an isopentyl group, a cyclopentyl group, a cyclobutylmethyl group and a 1-ethylpropyl group.

The alkoxyimino group having 1 to 5 carbon atoms means an imino group substituted with a straight, branched or cyclic alkoxy group, and examples thereof are a methoxyimino group, an ethoxyimino group, a propoxyimino group, an isopropoxyimino group, a butoxyimino group, an isobutoxyimino group, a tert-butoxyimino group, a cyclopropylmethoxyimino group, a pentyloxyimino group and an isopentyloxyimino group.

The alkylidene group having 1 to 5 carbon atoms means a straight, branched or cyclic alkylidene group, and examples thereof are a methylene group, an ethylidene group, a propylidene group, an isopropylidene group, a butylidene group, an isobutylidene group, a cyclopropylmethylene group and a pentylidene group.

The alkyl group having 1 to 10 carbon atoms which is optionally substituted means a straight, branched or cyclic alkyl group having 1 to 10 carbon atoms which is substituted or unsubstituted, and examples thereof are a methyl group, an ethyl group, a propyl group, an isopropyl group, a butyl group, an isobutyl group, a sec-butyl group, a tert-butyl group, a pentyl group, an isopentyl group, a hexyl group, a heptyl group, an octyl group, a nonyl group, a decyl group, a cycloalkyl group having 3 to 10 carbon atoms (e.g., a cyclopropyl group, a cyclobutyl group, a cyclopentyl group, a cyclobutylmethyl group, a cyclohexyl group, a cycloheptyl group or a cyclooctyl group), a cycloalkenyl group having 4 to 8 carbon atoms (e.g., a cyclobutenyl group, a cyclopentenyl group, a cyclohexenyl group, a cycloheptenyl group or a cyclooctenyl group), a bicycloalkyl group having 5 to 10 carbon atoms which is optionally substituted (e.g., a bicyclopentyl group, a bicyclohexyl group, a bicycloheptyl group, a bicyclooctyl group, a bicyclononyl group or a bicyclodecyl group), a bicycloalkenyl group having 5 to 10 carbon atoms which is optionally substituted (e.g., a bicyclopentenyl group, a bicyclohexenyl group, a bicycloheptenyl group, a bicyclooctenyl group, a bicyclononenyl group or a bicyclodecenyl group), a bridged cyclic hydrocarbon group (e.g., an adamantyl group, a bornyl group, a norbornyl group, a pinanyl group, a thujyl group, a caryl group or a camphanyl group), and the alkyl group of which the hydrogen atom is substituted with at least one selected from the group consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), $-CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a straight or branched alkyl group having 1 to 5 carbon atoms or a benzyl group).

Examples of the substituted phenyl group of the optionally substituted phenyl group are a phenyl group substituted with at least one selected from the group consisting of a hydroxyl group and a straight or branched alkoxy group having 1 to 5 carbon atoms (e.g., a 4-hydroxyphenyl group or a 3,4-dimethoxyphenyl group).

Examples of the substituted pyridyl group of the optionally substituted pyridyl group (e.g., a pyridin-2-yl group) are a pyridyl group substituted with at least one selected from the group consisting of a cyano group, a nitro group, a halogen atom and an aminocarbonyl group (e.g., a 5-cyanopyridin-2-yl group, a 5-nitropyridin-2-yl group, a chloropyridin-2-yl group or a 5-aminocarbonylpyridin-2-yl group).

Examples of the hydroxyalkyl group having 1 to 5 carbon atoms are a hydroxymethyl group, a 1-hydroxyethyl group, a 2-hydroxyethyl group, a 1-hydroxypropyl group, a 2-hydroxypropyl group, a 3-hydroxypropyl group, a 1-(hydroxymethyl)ethyl group, a 1-hydroxy-1-methylethyl group, a 4-hydroxybutyl group and a 5-hydroxypentyl group.

Examples of the an alkylthio group having 1 to 5 carbon atoms are a methylthio group, an ethylthio group, a propylthio group, an isopropylthio group, a butylthio group, a tert-butylthio group and a pentylthio group.

The alkenyl group having 2 to 10 carbon atoms which is optionally substituted means a straight, branched or cyclic alkenyl group having 2 to 10 carbon atoms which is substituted or unsubstituted, and examples thereof are alkenyl groups (e.g., a vinyl group, an allyl group, a propenyl group, an isopropenyl group, a butenyl group, an isobutenyl group, a pentenyl group, a hexenyl group, a heptenyl group, an octenyl group, a cyclopentenyl group or a cyclohexenyl group) and the alkenyl group of which the hydrogen atom is substituted with at least one group selected from the group consisting of a halogen atom, a hydroxyl group, a carboxyl group, an amino group, an aminocarbonyl group and a straight or branched alkoxy group having 1 to 5 carbon atoms.

The cycloalkyl group having 3 to 8 carbon atoms which is optionally substituted means a cycloalkyl group which is substituted or unsubstituted, and examples thereof are a cycloalkyl group (e.g., a cyclopropyl group, a cyclobutyl group, a cyclopentyl group, a cyclohexyl group, a cycloheptyl group or a cyclooctyl group) and the cycloalkyl group of which the hydrogen atom is substituted with at least one selected from the group consisting of a halogen atom, a hydroxyl group, a carboxyl group, an amino group, an aminocarbonyl group, a straight or branched alkyl group having 1 to 5 carbon atoms and a straight or branched alkoxy group having 1 to 5 carbon atoms.

The cycloalkenyl group having 4 to 8 carbon atoms which is optionally substituted means a cycloalkenyl group which is substituted or unsubstituted, and examples thereof are a cylcoalkenyl group (e.g., a cyclobutenyl group, a cyclopentenyl group, a cyclohexenyl group, a cycloheptenyl group or a cyclooctenyl group) and the cycloalkenyl group of which the hydrogen atom is substituted with at least one selected from the group consisting of a halogen atom, a hydroxyl group, a carboxyl group, an amino group, an aminocarbonyl group, a straight or branched alkyl group having

1 to 5 carbon atoms and a straight or branched alkoxy group having 1 to 5 carbon atoms.

The bicycloalkyl group having 5 to 10 carbon atoms which is optionally substituted means a bicycloalkyl group which is substituted or unsubstituted, and examples thereof are a bicycloalkyl group (e.g., a bicyclopentyl group, a bicyclohexyl group, a bicycloheptyl group, a bicyclooctyl group, a bicyclononyl group or a bicyclodecyl group) and the bicycloalkyl group of which the hydrogen atom is substituted with at least one selected from the group consisting of a halogen atom, a hydroxyl group, a carboxyl group, an amino group, an aminocarbonyl group, a straight or branched alkyl group having 1 to 5 carbon atoms and a straight or branched alkoxy group having 1 to 5 carbon atoms.

The bicycloalkenyl group having 5 to 10 carbon atoms which is optionally substituted means a bicycloalkenyl group which is substituted or unsubstituted, and examples thereof are bicycloalkenyl groups (e.g., a bicyclopentenyl group, a bicyclohexenyl group, a bicycloheptenyl group, a bicyclooctenyl group, a bicyclononenyl group or a bicyclodecenyl group) and the bicycloalkenyl group of which the hydrogen atom is substituted with at least one group selected from the group consisting of a halogen atom, a hydroxyl group, a carboxyl group, an amino group, an aminocarbonyl group, a straight or branched alkyl group having 1 to 5 carbon atoms and a straight or branched alkoxy group having 1 to 5 carbon atoms.

The cyclic amino group having 2 to 10 carbon atoms which is optionally substituted means a cyclic amino group which has at least one nitrogen atom and has optionally at least one of an oxygen atom and a sulfur atom on the ring, and is substituted or unsubstituted, and examples thereof are a cyclic amino group (e.g., an aziridyl group, an azetidyl group, a pyrrolidyl group, an imidazolidyl group, an oxazolidyl group, a thiazolidyl group, a piperidyl group, a morpholinyl group, an azabicycloheptyl group or an azabicyclooctyl group), the cyclic amino group which is condensed with a benzene ring or a pyridine ring, and the cyclic amino group (including those which are condensed with a benzene ring or a pyridine ring) of which the hydrogen atom is substituted with at least one group selected from the group consisting of a halogen atom, a hydroxyl group, an amino group, a straight or branched alkyl group having 1 to 5 carbon atoms, and $-OR^{15}$ (wherein $R^{15}$ is a straight or branched alkyl group having 1 to 5 carbon atoms, an aminocarbonylmethyl group or a benzyl group).

Examples of the pharmaceutically acceptable salt are salts with mineral acids such as sulfuric acid, hydrochloric acid, hydrobromic acid or phosphoric acid, and salts with organic acids such as acetic acid, oxalic acid, lactic acid, tartaric acid, fumaric acid, maleic acid, trifluoroacetic acid or methanesulfonic acid.

Preferred compounds of the present invention are shown as follows:

In view of DPP IV inhibition activity, $R^1$ is preferably a halogen atom, and especially preferably a fluorine atom. $R^2$ is preferably a hydrogen atom or a halogen atom, and especially preferably a hydrogen atom.

In Formula (1) or (2), when Y is $-CH_2-$, Z is preferably an alkyl group having 1 to 10 carbon atoms which is optionally substituted with at least one selected from the group consisting of a hydroxyl group, an alkoxy group having 1 to 5 carbon atoms, a hydroxyalkyl group having 1 to 5 carbon atoms, an optionally substituted phenyl group and $-NHR^{11}$ (wherein $R^{11}$ is an optionally substituted pyridyl group) . In this case, Z is preferably a branched or cyclic alkyl group having 4 to 10 carbon atoms which is optionally substituted with at least one selected from the group consisting of a hydroxyl group, a hydroxyalkyl group having 1 to 5 carbon atoms and an alkoxy group having 1 to 5 carbon atoms, and especially preferably a branched alkyl group having 4 to 10 carbon atoms which is optionally substituted with at least one selected from the group consisting of a hydroxyl group and a hydroxyalkyl group having 1 to 5 carbon atoms, a cycloalkyl group having 4 to 10 carbon atom or an adamantyl group, and most preferably a tert-butyl group, a (1-hydroxymethy)cyclopentyl group or a (2-hydroxy-1,1-dimethyl)ethyl group.

In Formula (1) or (2), it is preferable that when Y is $-CR^5R^6-$ (wherein $R^5$ is a hydrogen atom, $R^6$ is an optionally substituted alkyl group having 1 to 10 carbon atoms) or $-CR^7R^8-CR^9R^{10}-$ (wherein $R^8$ and $R^{10}$ are each a hydrogen atom, and $R^7$ and $R^9$ together with the carbon atom to which they are attached form a cycloalkyl group having 3 to 8 carbon atoms), Z is H or $-CH_3$.

In this case, it is further preferable that when Y is $-CR^5R^6-$ {wherein $R^5$ is a hydrogen atom, $R^6$ is a branched or cyclic alkyl group having 3 to 6 carbon atoms which is optionally substituted with at least one selected from the group consisting of a hydroxyl group and $-OR^{14}$ (wherein $R^{14}$ is a straight or branched alkyl group having 1 to 5 carbon atoms or a benzyl group)}, Z is a hydrogen atom. It is further preferable that when Y is $-CR^5R^6-$ (wherein $R^5$ is a hydrogen atom, and $R^6$ is a branched or cyclic alkyl group having 3 to 6 carbon atoms), Z is a hydrogen atom, and it is especially preferable that when Y is $-CH[CH(CH_3)_2]-$, $-CH[C(CH_3)_3]-$ or $-CH[CH(CH_3)CH_2CH_3]-$, Z is a hydrogen atom.

In Formula (1) or (2), preferred examples of the optionally substituted cyclic amino group having 2 to 10 carbon atoms which is formed by Y and Z together with the nitrogen atom to which they are attached, are a pyrrolidyl group, a piperidyl group and a cyclic amino group which is formed by condensing a pyrrolidyl group or a piperidyl group with a benzene ring, and preferred substituent thereof includes a hydroxyl group or $-OR^{15}$ (wherein $R^{15}$ is defined as above).

The compounds of the present invention can inhibit dipeptidyl peptidase IV, and therefore, they increase insulin activity, improve glucose metabolism, and can contribute to inhibition of neuropeptide Y metabolism, inhibition of T-cell activity, inhibition of adhesion of cancer cells to the endothelium and prevention of invasion of HIV virus into

lymphocytes.

Accordingly, the present invention is to provide a pharmaceutical preparation for preventing or treating diseases or conditions capable of being improved by inhibition of dipeptidyl peptidase IV, for example, diabetes mellitus (especially the type 2), immune diseases, arthritis, obesity, osteoporosis, conditions of glucose tolerance, benign prostatic hypertrophy or skin diseases.

The pharmaceutical preparation for immune diseases includes immunosuppresors for tissue transplantation, for example, cytokine-release inhibitors in various autoimmune diseases such as inflammatory enteritis, multiple sclerosis or chronic rheumatoid arthritis (RA), drugs useful for preventing or treating AIDS due to prevention of invasion of HIV into T-cells, drugs for metastasis obviation, especially, metastasis obviation of breast or prostatic cancer into lung.

The pharmaceutical preparation of present invention can be administered systemically or locally, or orally or parentelly such as rectally, subcutaneously, intramuscularly, intravenously or percutaneusly.

For use of the compound of the present invention as a pharmaceutical preparation, any dosage form can be properly selected as necessary from solid compositions, liquid compositions and other compositions. The pharmaceutical preparation of the present invention can be produced by combining the compound of the present invention with pharmaceutically acceptable carriers. Specifically, tablets, pills, capsules, granules, powders, fine powders, solutions, emulsions, suspensions or injections can be produced by adding convenient excipients, fillers, binders, disintegrators, coating agents, sugar coating agents, pH modulators, solublizing agents, or aqueous or non-aqueous solvents according to the conventional pharmaceutical preparation techniques. Excipients and fillers include lactose, magnesium stearate, starch, talc, gelatin, agar, pectin, arabic gum, olive oil, sesame oil, cacao butter, ethylene glycol and other conventional materials.

Furthermore, the compounds of the present invention can be formulated into the form of an inclusion compound with α-, β- or γ-cyclodextrin, or methylated cyclodextrin.

A dose of the compound of the present invention varies depending upon disease, conditions, body weight, age, sex, administration route, but the dose for adult is preferably from about 1 to about 1000 mg/kg of body weight/day for oral administration, and specifically preferably from about 10 to about 200 mg/kg of body weight/day, and may be given at once or by dividing.

The compounds of Formula (1) can be prepared by the general preparation processes.

[General Preparation Process]

[Reaction Scheme 1]

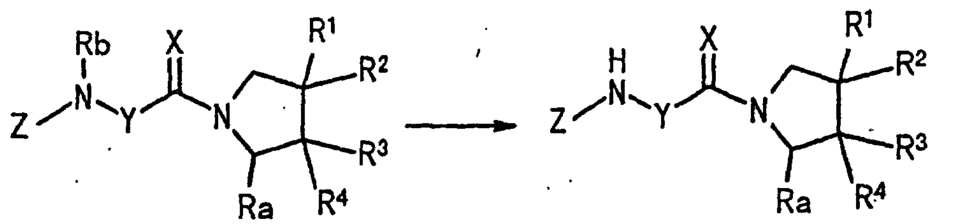

wherein X, Y, Z, $R^1$, $R^2$, $R^3$ and $R^4$ are defined as above, Ra is a cyano group, an aminocarbonyl group or an alkoxycarbonyl group, and Rb is a protecting group of an amino group.

The deprotection can be carried out by the method described in Protective Groups in Organic Synthesis by Theodora W. Greene and Peter G. M. Wu Ts.

For example, the compound wherein Rb is a group to be deprotected with an acid (e.g., a tert-butoxycarbonyl group, a trityl group or an o-nitrobenzenesulfenyl group) can be deprotected using an acid such as hydrochloric acid, sulfuric acid, trifluoroacetic acid, p-toluenesulfonic acid or methanesulfonic acid. In this case, the deprotection can be carried out using the acid which is diluted with or dissolved in an organic solvent or water. The reaction can be carried out at a temperature of -50 to 50°C. Examples of the organic solvent are ethanol, methanol, tetrahydrofuran, N,N-dimethylformamide, dichloromethane, chloroform and 1,2-dichloroethane. Furthermore, for example, the compound wherein Rb is a group to be deprotected by hydrogenolysis (e.g., a benzyloxycarbonyl group) can be deprotected by hydrogenolysis using a metal catalyst (e.g., palladium). The solvent to be used includes a reaction-inert solvent (e.g., ethanol, methanol, tetrahydrofuran or ethyl acetate). The reaction can be carried at a temperature of 0 to 100°C.

Furthermore, this reaction can be also carried out using a hydrogen gas or using a combination of reagents (e.g., formic acid - ammonium formate). For another example, the compound wherein Rb is a protective group to be deprotected by a base (e.g., a fluorenyloxycarbonyl group) can be deprotected using a base (e.g., diethylamine, piperidine, ammonia, sodium hydroxide or potassium carbonate). These bases can be used directly or after diluting with, dissolving or suspending in a solvent. In this case, examples of the solvent to be used are water, ethanol, methanol, tetrahydrofuran, N,N-dimethylformamide, dichloromethane, chloroform and 1,2-dichloroethane. The reaction can be carried out at a temperature of 0 to 100°C. In addition, the compound wherein Rb is a group to be deprotected by a metal catalyst (e.g., an allyloxycarbonyl group) can be deprotected using, for example, tetrakis(triphenylphoshine)palladium as a catalyst or a reagent in a reaction-inert solvent (e.g., dichloromethane, chloroform or tetrahydrofuran). The reaction can be carried out at a temperature of 0 to 100°C.

[Reaction Scheme 2]

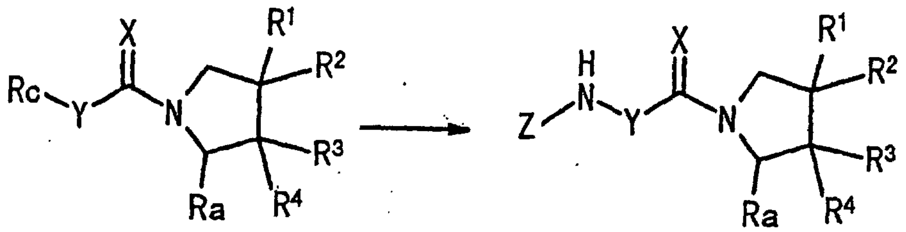

wherein X, Y, Z, $R^1$, $R^2$, $R^3$, $R^4$ and Ra are defined as above, and Rc is a leaving group (e.g., a halogen atom or a sulfonyloxy group) or a group capable of being converted into a leaving group.

For example, the compound wherein Rc is a leaving group (e.g., a chlorine atom, a bromine atom, an iodine atom, a methanesulfonyloxy group or a p-toluenesulfonyloxy group) can be subjected to a substitution reaction using a primary amine ($Z\text{-}NH_2$)(e.g., ethylamine, isopropylamine, tert-butylamine, benzylamine, a substituted benzylamine, phenethylamine, a substituted phenethylamine or a 2-(substituted pyridylamino)ethylamine). In this case, the amine may be used in an excess amount, or alternatively a base may be further added. Examples of the base to be added are an amine (e.g., triethylamine or diisopropylethylamine) or an inorganic base (e.g., potassium carbonate). If necessary, for example, sodium iodide may be added for accelerating the reaction. The reaction solvent includes a reaction-inert solvent such as N,N-dimethylformamide, tetrahydrofuran, dioxane, dichloromethane or chloroform. The reaction can be carried out at a temperature of 0 to 100°C.

An example of the group, presented by Rc, capable of being converted into a leaving group, is a hydroxyl group, in this case, the above-mentioned reaction can be carried out after chlorination, bromination, iodination, methanesulfonation, p-toluenesulfonation or the like. Examples of chlorination are a method using carbon tetrachloride and triphenylphosphine, a method using thionyl chloride or phosphorus oxychloride and a method for substituting a leaving group with lithium chloride or the like after forming the leaving group with tosyl chloride, etc. These reactions can be carried out using a reaction-inert solvent such as tetrahydrofuran, dioxane, dichloromethane, chloroform or N,N-dimethylformamide, at a temperature of -50 to 100°C. An example of bromination is a method using carbon tetrabromide and triphenylphosphine. This reaction can be carried out by using a reaction-inert solvent such as tetrahydrofuran, dioxane, dichloromethane, chloroform or N,N-dimethylformamide, at a temperature of -50 to 50°C. An example of iodination is a method using iodine, triphenylphosphine and imidazole. This reaction can be carried out by using a reaction-inert solvent such as tetrahydrofuran, dioxane, dichloromethane, chloroform or N,N-dimethylformamide, at a temperature of -50 to 100°C. Methanesulfonation and p-toluenesulfonation are each carried out by a method using methanesulfonyl chloride and p-toluenesulfonyl chloride, respectively. In these cases, a suitable base can be optionally added. Examples of the base to be added are an amine (e.g., triethylamine or diisopropylethylamine) and an inorganic base (e.g., potassium carbonate). As the reaction solvent can be used a reaction-inert solvent (e.g., N,N-dimethylformamide, tetrahydrofuran, dioxane, dichloromethane, chloroform or 1,2-dichloroethane), and the reaction can be carried out at a temperature of -50 to 50°C.

[Reaction Scheme 3]

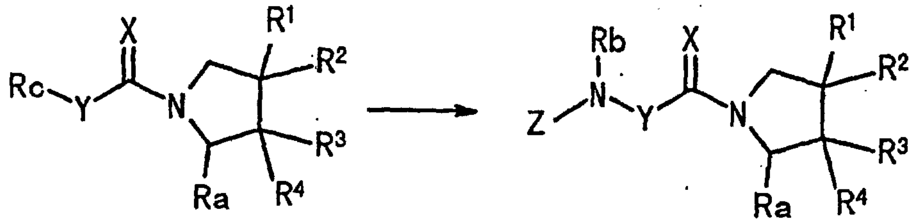

wherein X, Y, Z, $R^1$, $R^2$, $R^3$, $R^4$, Ra, Rb and Rc are defined as above.

For example, the compound wherein Rc is a leaving group (e.g., a chlorine atom, a bromine atom, iodine atom, a methanesulfonyloxy group or a p-toluenesufonyloxy group) can be subjected to substitution reaction using a compound represented by Z-NH-Rb (wherein Z and Rb are defined as above). In this case, for example, sodium hydride, potassium tert-butoxide, n-butyl lithium or lithium diisopropylamide can be used as a base. As a solvent can be used N,N-dimethylformamide, tetrahydrofuran or dioxane. The reaction can be carried out at a temperature of -50 to 50°C.

An example of the group, presented by Rc, capable of being converted into a leaving group, is a hydroxyl group. In this case, the above-mentioned reaction can be carried out after chlorination, bromination, iodination, methanesulfonation or p-toluenesulfonation as illustrated in Reaction Scheme 2.

[Reaction Scheme 4]

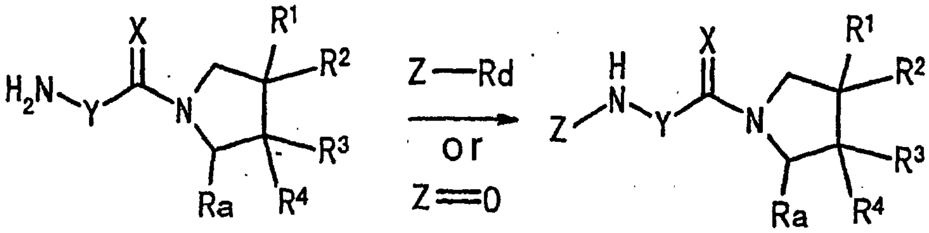


wherein X, Y, Z, $R^1$, $R^2$, $R^3$, $R^4$ and Ra are defined as above, and Rd is a leaving group such as a halogen atom or a sulfonyloxy group.

When Z-Rd is used, the amino group of the material can be reacted with Z-Rd to give the object compound. For example, the compound wherein Rd is a chlorine atom, a bromine atom, an iodine atom, a methanesulfonyloxy group, a p-toluenesulfonyloxy group or the like can be reacted with an amino compound of the material in the presence of a suitable base. Examples of the base to be added are amines (e.g., triethylamine or diisopropylethylamine) and inorganic bases (e.g., potassium carbonate). Examples of the reaction solvent are N,N-dimethylformamide, tetrahydrofuran and dioxane. The reaction can be carried out at a temperature of 0 to 100°C.

In addition, when Z=O (aldehyde or ketone compound) is used, the reaction can be carried out using a primary amino group of the material under the conditions for a suitable reduction method. The reduction method to be used includes a hydrogenation using a reductant (e.g., sodium borohydride or sodium cyanoborohydride) or palladium. Examples of the solvent to be used are reaction-inert solvents (e.g., ethanol, methanol, tetrahydrofuran, dioxane or water). The reaction can be carried out at a temperature of -20 to 100°C.

[Reaction Scheme 5]

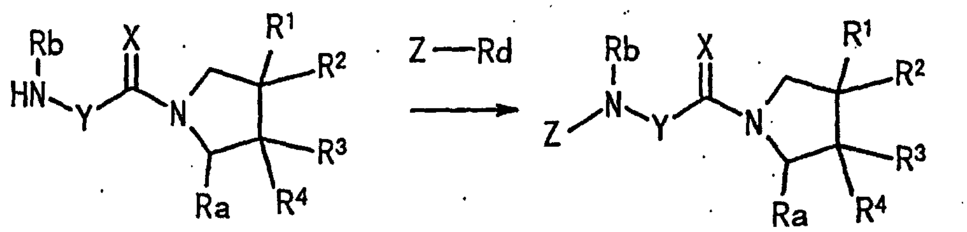

wherein X, Y, Z, $R^1$, $R^2$, $R^3$, $R^4$, Ra, Rb and Rd are defined as above.

The protected amino group of the material is reacted with Z-Rd to give the object compound. In this case, the reaction can be carried out by adding a suitable base, examples of which are sodium hydride, potassium tert-butoxide, n-butyl lithium and lithium diisopropylamide. Examples of the reaction solvent are N,N-dimethylformamide, tetrahydrofuran and dioxane. The reaction can be carried out at a temperature of -50 to 50°C.

[Reaction Scheme 6]

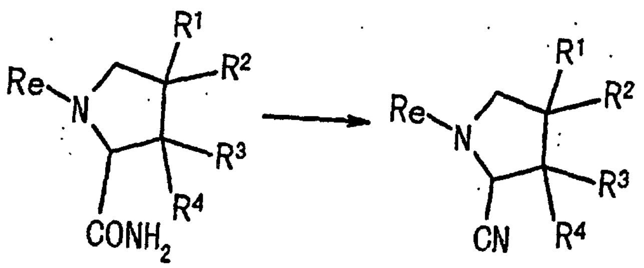

wherein Re is -C(=X)-Y-NH-Z, -C(=X)-Y-N(Rb)-Z, -C(=X)-Y-Rc or Rb, and $R^1$, $R^2$, $R^3$, $R^4$, X, Y, Z, Rb and Rc are defined as above.

The aminocarbonyl group can be converted into a cyano group by a general dehydration, an example of which is a method using trifluoroacetic anhydride. The solvent to be used herein includes a reaction-inert solvent such as dichloromethane, chloroform, 1,2-dichloroethane, tetrahydrofuran, dioxane or N,N-dimethylformamide. In this case, a base (e.g., triethylamine, diisopropylethylamine, sodium bicarbonate or potassium carbonate) can be used if necessary. The reaction can be carried out at a temperature of -50 to 50°C.

Another example is a method using phosphorus oxychloride. In this case, the solvent to be used herein includes dichloromethane, chloroform, 1,2-dichloroethane, tetrahydrofuran, dioxane or pyridine. They can be used alone or in combination with at least two solvents thereof. This reaction may be carried out by adding imidazole, etc., at a temperature of -50 to 50°C.

Still another example is a method using cyanuric chloride and N,N-dimethylformamide. In this case, the solvent to be used herein includes dichloromethane, chloroform, 1,2-dichloroethane, tetrahydrofuran, dioxane or pyridine. They can be used alone or in combination with at least two solvents thereof. This reaction can be carried out at a temperature of -50 to 50°C.

[Reaction Scheme 7]

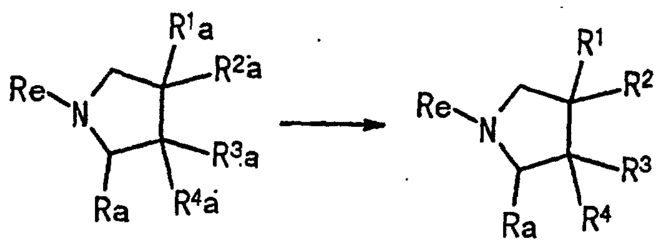

wherein Re, $R^1$, $R^2$, $R^3$, $R^4$ and Ra are defined as above, and $R^1a$, $R^2a$, $R^3a$ and $R^4a$ are the same as $R^1$, $R^2$, $R^3$ and $R^4$, respectively, or the groups capable of being converted into $R^1$, $R^2$, $R^3$ and $R^4$, respectively.

An example of modification of the pyrrolidine ring is a conversion of the substituent(s). For example, the compound wherein one of $R^1a$, $R^2a$, $R^3a$ and $R^4a$ is a hydroxyl group is subjected to halogenation to give a fluorine compound, a chlorine compound or a bromine compound or the like. In detail, examples of fluorination are methods using diethylaminosulfur trifluoride, dimethylsulfur trifluoride, etc. These reactions are started at a temperature of -78°C to room temperature and achieved by maintaining a temperature at room temperature to 50°C using a reaction-inert solvent such as tetrahydrofuran, dioxane, dichloromethane, chloroform, 1,2-dichloroethane or toluene. Another example of fluorination is a method for converting a hydroxyl group into a leaving group, followed by conversion into a fluorine atom. The conversion into the leaving group is carried out by the same method as illustrated in Reaction Scheme 2. After the conversion into the leaving group, the method for converting into a fluorine atom includes methods for reaction with tetrabutylammonium fluoride, caesium fluoride, or the like. These methods can be carried out using a reaction-inert solvent such as tetrahydrofuran, dioxane, dichloromethane, chloroform, N,N-dimethylformamide or water at a temperature of -50 to 100°C.

Examples of chlorination are a method using carbon tetrachloride and triphenylphosphine, a method using thionyl chloride and phosphorus oxychloride and a method for converting into a leaving group using tosyl chloride, etc. and substituting it with lithium chloride, etc. These reactions can be carried out using a reaction-inert solvent such as tetrahydrofuran, dioxane, dichloromethane, chloroform and N,N-dimethylformamide at a temperature of -50 to 100°C.

Still another example is a method for sterically inverting a hydroxyl group, example of which is a Mitsunobu reaction. In this reaction, the hydroxyl group is reacted with diethyl azodicarboxylate, triphenylphosphine and a carboxylic acid such as acetic acid to give a sterically inverted ester, which is then hydrolyzed to give a inverted hydroxyl group. This reaction is carried out using a reaction-inert solvent such as tetrahydrofuran, dioxane, dichloromethane, chloroform or N,N-dimethylformamide at a temperature of -50 to 50°C.

The compound wherein $R^1$ and $R^2$, or $R^3$ and $R^4$ together form an oxo can be synthesized by an oxidation using a compound wherein one of $R^1a$, $R^2a$, $R^3a$ and $R^4a$ is a hydroxyl group. Examples of the oxidation are a method using a chromate oxidant (e.g., pyridinium chlorochromate or pyridinium dichromate) and a DMSO oxidation method using activating agents (e.g., dimethyl sulfoxide and oxalyl chloride). For example, the reaction using pyridinium chlorochromate can be carried out using a reaction-inert solvent such as dichloromethane, chloroform, N,N-dimethylformamide, tetrahydrofuran or dioxane at a temperature of 0 to 50°C.

[Reaction Scheme 8]

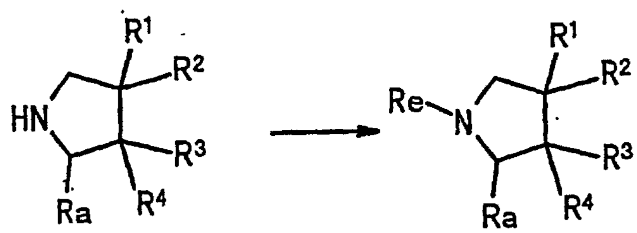

wherein $R^1$, $R^2$, $R^3$, $R^4$, Re and Ra are defined as above.

A 1-H-pyrrolidine derivative or a salt thereof is subjected to condensation to give an amide compound, a thioamide compound or a carbamate compound. For example, amidation can be carried out using an acyl halide (e.

g., an acyl chloride or an acyl bromide) in a reaction-inert solvent such as dichloromethane, chloroform, 1,2-dichloroethane, tetrahydrofuran, dioxane, toluene or ethyl acetate. In this case can be used a base, examples of which are amines (e.g., triethylamine or diisopropylethylamine), organic acid salts (e.g., sodium 2-ethylhexanoate or potassium 2-ethylhexanoate) and inorganic bases (e.g., potassium carbonate). These reactions can be carried out at a temperature of -50 to 100°C. Another amidation can be carried out using an activating ester such as 1-benzotriazolyl ester or succinimidyl ester in a reaction solvent (e.g., dichloromethane, chloroform, 1,2-dichloroethane, N,N-dimethylformamide, tetrahydrofuran, dioxane, toluene or ethyl acetate). The reaction can be carried out at a temperature of -50 to 50°C.

Still another amidation can be carried out by using a carboxylic acid and a condensing agent for dehydration. Examples of the condensing agent for dehydration are 1-ethyl-3-(3-dimethylaminopropyl)carbodiimide·hydrochloride, dicyclohexylcarbodiimide, diphenylphosphoryl azide and carbonyldiimidazole. If necessary, an activating agent (e.g., 1-hydroxybenzotriazole or hydroxysuccinimide) can be used. Examples of the reaction solvent are dichloromethane, chloroform, 1,2-dichloroethane, N,N-dimethylformamide, tetrahydrofuran, dioxane, toluene and ethyl acetate. In this case can be used a base, examples of which are amines (e.g., triethylamine or diisopropylethylamine), organic acid salts (e.g., sodium 2-ethylhexanoate or potassium 2-ethylhexanoate) and inorganic bases (e.g., potassium carbonate). The reaction can be carried out at a temperature of -50 to 50°C.

Furthermore, for example, amidation can be carried out by using a mixed acid anhydride obtained from a carboxylic acid and a chlorocarboxylate ester. The solvent for the reaction includes reaction-inert solvents such as tetrahydrofuran, dioxane, dichloromethane, chloroform, N,N-dimethylformamide, toluene or ethyl acetate. In this case can be used a base, examples of which are amines (e.g., triethylamine or diisopropylethylamine), organic acid salts (e.g., sodium 2-ethylhexanoate or potassium 2-ethylhexanoate) and inorganic bases (e.g., potassium carbonate). The reaction can be carried out at a temperature of -50 to 50°C.

Protection of the amino group can be carried out using di-tert-butyldicarboxylate, benzyloxycarbonyl chloride or fluorenylmethoxycarbonyl chloride in the presence of a suitable base. Examples of the base are amines (e.g., triethylamine or diisopropylethylamine) and inorganic bases (e.g., potassium carbonate). The solvents in these reactions include reaction-inert solvents such as tetrahydrofuran, dioxane, dichloromethane, chloroform, N,N-dimethylformamide, toluene, ethyl acetate or water. These reactions can be carried out at a temperature of -50 to 50°C.

[Reaction Scheme 9]

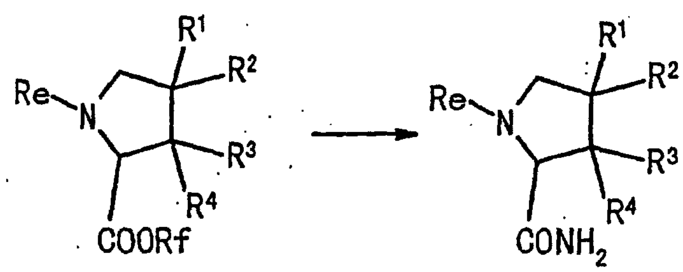

wherein $R^1$, $R^2$, $R^3$, $R^4$ and Re are defined as above, and Rf is a hydrogen atom, a lower alkyl group, a benzyl group, an allyl group, etc.

The method is a conversion of a carboxyl group, a salt thereof or an ester thereof into an aminocarbonyl group. When the compound wherein COORf is a carboxyl group or a salt thereof is used as a material, ammonia can be used under an ordinary amidation condition for the synthesis. An example of the amidation is a method for converting a carboxyl group or a salt thereof into an acid chloride using thionyl chloride, phosphorus oxychloride or oxalyl chloride, followed by condensation with ammonia. Examples of the solvent in these reactions are reaction-inert solvents such as tetrahydrofuran, dioxane, dichloromethane, chloroform, 1,2-dichloroethane, N,N-dimethylformamide, toluene or ethyl acetate. These reactions can be carried out at a temperature of -50 to 50°C.

Another example of the amidation is a method using a condensing agent for dehydration and ammonia. In this reaction is used a condensing agent such as 1-ethyl-3-(3-dimethylaminopropyl)carbodiimide hydrochloride, dicyclohexylcarbodiimide, diphenylphosphoryl azide or carbonyldiimidazole. If necessary, an activating agent (e.g., 1-hydroxybenzotriazole or hydroxysuccinimide) can be added. Examples of the solvent in these reactions are reaction-inert solvents such as tetrahydrofuran, dioxane, dichloromethane, chloroform, 1,2-dichloroethane, N,N-dimethylformamide, toluene, ethyl acetate or acetonitrile. These reactions can be carried out at a temperature of -50 to 50°C.

Still another example of the amidation is a method using a mixed acid anhydride (obtained from a carboxylic

acid and a chlorocarbonate ester) and ammonia. Examples of the solvent in the reaction are reaction-inert solvents such as tetrahydrofuran, dioxane, dichloromethane, chloroform, 1,2-dichloroethane, N,N-dimethylformamide, toluene or ethyl acetate. These reactions can be carried out at a temperature of -50 to 50°C.

Conversion of COORf (which is an ester) of the compound into an aminocarbonyl group can be carried out by a direct reaction with ammonia, or can be carried out by conversion of the ester into a carboxylic acid or a salt thereof, followed by conversion of the carboxylic acid into an aminocarbonyl group according to the above-mentioned method. The conversion of an ester into a carboxylic acid or a salt thereof is carried out according to the method described in Protective Groups in Organic Synthesis by Theodora W. Greene and Peter G. M. Wu Ts. The conversion by the direct reaction with ammonia is carried out using ammonia gas or aqueous ammonia in a solvent (e.g., water, methanol, ethanol, tetrahydrofuran, dioxane, dichloromethane, chloroform, N,N-dimethylformamide or toluene) or without a solvent at a temperature of 0 to 100°C and, if necessary, with sealing for preventing volatilization of ammonia.

[Reaction Scheme 10]

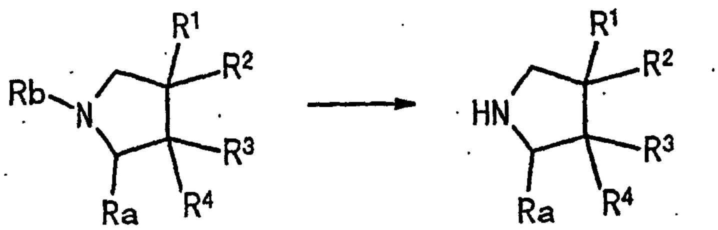

wherein $R^1$, $R^2$, $R^3$, $R^4$, Ra and Rb are defined as above.

Rb (which is a protecting group of an amino group) can be deprotected, for example, by a method described in Reaction Scheme 1. The resulting amine can be obtained as a base or an acid salt. Examples of the suitable acid to be used are hydrochloric acid, sulfuric acid, trifluoroacetic acid, p-toluenesulfonic acid, methanesulfonic acid and acetic acid.

The present invention is illustrated in more detail by the following reference examples, examples and experiments, however, being not limited thereto.

Reference Example 1

Synthesis of (2S,4S)-1-(tert-butoxycarbonyl)-4-fluoropyrrolidine-2-carboxylic acid

Referring to Tetrahedron Letter 39(10), 1169 - 1172 (1998), the title compound (4.5 g) which is the material of Example 1(1) was obtained from methyl (2S,4R)-1-(tert-butoxycarbonyl)-4-hydroxypyrrolidine-2-carboxylate (6.8 g) by two steps.

Reference Example 2

Synthesis of (2S,4R)-1-(tert-butoxycarbonyl)-4-fluoropyrrolidine-2-carboxylic acid

Referring to Tetrahedron Letter 39(10), 1169 - 1172 (1998), the title compound (370 mg) which is the material of Example 3(1) was obtained from methyl (2S,4S)-1-(tert-butoxycarbonyl)-4-hydroxypyrrolidine-2-carboxylate (500 mg) by two steps.

Reference Example 3

Synthesis of (2S,4R)-2-(aminocarbonyl)-1-(tert-butoxycarbonyl)-4-hydroxypyrrolidine

In dioxane (50 mL) were suspended (2S,4R)-1-(tert-butoxycarbonyl)-4-hydroxypyrrolidine-2-carboxylic acid (2.31 g) and 1-hydroxybenzotriazole monohydrate (1.51 g), and then 1-(3,3-dimethylaminopropyl)-3-ethylcarbodiimide hydrochloride (2.11 g) and 25% aqueous ammonia (0.68 mL) were added with ice-cooling. The temperature was gradually raised to room temperature, and then, the mixture was stirred overnight. The reaction solution was concentrated under reduced pressure, and the resulting residue was purified by a silica gel column chromatography (developing

solvent; chloroform : methanol =100:3 - 100:7) to give the title compound (2.19 g) as a colorless powder.

Example 1

Synthesis of (2S,4S)-1-[(2S,3S)-2-amino-3-methylpentanoyl]-2-cyano-4-fluoropyrrolidine hydrochloride

(1) Synthesis of (2S,4S)-2-(aminocarbonyl)-1-(tert-butoxycarbonyl)-4-fluoropyrrolidine

In acetonitrile (50 mL) was dissolved (2S,4S)-1-(tert-butoxycarbonyl)-4-fluoropyrrolidine-2-carboxylic acid (4.5 g) obtained in Reference Example 1, then 1-hydroxybenzotriazole monohydrate (3.6 g) and 1-(3,3-dimethylaminopropyl)-3-ethylcarbodiimide hydrochloride (4.5 g) were added with ice-cooling. The temperature was gradually raised, and then, the mixture was stirred at room temperature overnight. The reaction solution was again ice-cooled, 25% aqueous ammonia (5 mL) was added, and stirring was continued with ice-cooling for 30 minutes then at room temperature for 30 minutes. To the reaction solution was added acetonitrile (50 mL), and an insoluble substance was removed by filtration. The filtrate was concentrated under reduced pressure and purified by a silica gel column chromatography (developing solvent; hexane : ethyl acetate =4:1 - 1:5). To the resulting residue was added hexane to give the title compound (4.2 g) as a colorless powder.

MS(ESI pos.)m/z: 255($[M+Na]^+$, (ESI neg.)m/z: 231($[M-H]^-$)

(2) Synthesis of (2S,4S)-2-(aminocarbonyl)-4-fluoropyrrolidine hydrochloride

In 4M-hydrochloric acid-dioxane (45 mL) was suspended (2S,4S)-2-(aminocarbonyl)-1-(tert-butoxycarbonyl)-4-fluoropyrrolidine (4.2 g) and, after stirring at room temperature for 2 hours, the reaction solution was concentrated under reduced pressure. To the residue was added toluene (50 mL), followed by further concentration under reduced pressure. This was repeated 3 times to give the title compound (3.1 g) as a colorless powder. This intermediate was used for the next reaction without purification.

(3) Synthesis of (2S,4S)-2-(aminocarbonyl)-1-[(2S,3S)-2-(fluorenylmethoxycarbonylamino)-3-methylpentanoyl]-4-fluoropyrrolidine

In tetrahydrofuran (40 mL) - N,N-dimethylformamide (10 mL) were dissolved (2S,4S)-2-(aminocarbonyl)-4-fluoropyrrolidine hydrochloride (2.4 g) and (2S,3S)-2-(fluorenylmethoxycarbonylamino)-3-methylpentanoic acid (5.1 g), and then 1-hydroxybenzotriazole monohydrate (2.6 g), 1-(3,3-dimethylaminopropyl)-3-ethylcarbodiimide hydrochloride (3.3 g) and diisopropylethylamine (2.5 mL) were added with ice-cooling, and the temperature was gradually raised, followed by stirring at room temperature overnight. The solution was concentrated under reduced pressure, and water was added to the resulting residue. The resulting powder was collected by filtration and purified by a silica gel column chromatography (developing solvent; hexane : ethyl acetate =4:1 - 1:4) to give the title compound (6.9 g) as a pale yellow amorphous substance.

MS(ESI pos.)m/z: 490($[M+Na]^+$).

(4) Synthesis of (2S,4S)-2-cyano-1-[(2S,3S)-2-(fluorenylmethoxycarbonylamino)-3-methylpentanoyl]-4-fluoropyrrolidine

In tetrahydrofuran (70 mL) was dissolved (2S,4S)-2-(aminocarbonyl)-1-[(2S,3S)-2-(fluorenylmethoxycarbonylamino)-3-methylpentanoyl]-4-fluoropyrrolidine (6.9 g), and trifluoroacetic anhydride (4.0 mL) was added with ice-cooling, followed by stirring with ice-cooling for 1.5 hours. The reaction solution was concentrated under reduced pressure, and the residue was purified by a silica gel column chromatography (developing solvent; hexane : ethyl acetate =8:1 - 3:2) to give the title compound (6.2 g) as a pale yellow amorphous substance.

MS(ESI pos.)m/z: 472($[M+Na]^+$)

(5) Synthesis of (2S,4S)-1-[(2S,3S)-2-amino-3-methylpentanoyl]-2-cyano-4-fluoropyrrolidine hydrochloride

In 1,2-dichloroethane (90 mL) was dissolved (2S,4S)-2-cyano-1-[(2S,3S)-2-(fluorenylmethoxycarbonylamino)-3-methylpentanoyl]-4-fluoropyrrolidine (6.2 g) and, after addition of diethylamine (10 mL) with ice-cooling, stirring was continued with ice-cooling for 30 minutes and then at room temperature for 5 hours. The solution was concentrated under reduced pressure, the residue was dissolved in a mixture of diethyl ether (100 mL), tetrahydrofuran (50 mL) and chloroform (50 mL), and then 4M hydrochloric acid - dioxane (4.0 mL) was added with ice-cooling. The resulting salt was collected by filtration and washed with diethyl ether. The resulting powder was purified by a silica gel column

chromatography (developing solvent; chloroform : methanol : 25% aqueous ammonia =40:1:0.1 - 25:1:0.1). The resulting residue was dissolved in chloroform and, after addition of 4M hydrochloric acid-dioxane (4.0 mL) with ice-cooling, the resulting salt was collected by filtration, washed with chloroform and dried under reduced pressure to give the title compound (2.9 g) as a colorless powder.

MS(ESI pos.)m/z: 228($[M+H]^+$), 250($[M+Na]^+$), (ESI neg.)m/z: 262($[M+CL]^-$).

$^1$H-NMR(DMSO-$d_6$,500MHz)δ; 8.59(3H,br s), 5.54(1H,br d, J=52.1Hz), 5.06(1H,d,J=9.4Hz), 4.07-3.77(3H,m), 2.55-2.34(2 H, m), 1.88(1H,m), 1.61(1H,m), 1.17(1H,m), 0.94(3H,d,J=6.7Hz ),0.88(3H,t,J=7.3Hz).

Example 2

Synthesis of (2S,4S)-1-[(2S,3S)-2-amino-3-methylpentanoyl]-2-cyano-4-fluoropyrrolidine hydrobromide.

In ethanol (1 mL) was dissolved (2S,4S)-1-[(2S,3S)-2-amino-3-methylpentanoyl]-2-cyano-4-fluoropyrrolidine (0.5 g) obtained in Example 1, and the solution was added to an ice-cooled ethanol solution (2 mL) of 48% hydrobromic acid (0.26 mL). Further, ethanol (2 mL) and pentane (3 mL) were added and stirred, and the precipitated crystals were collected by filtration. The resulting crystals were dissolved in methanol (1.75 mL) and added to an ice-cooled 2-propanol (14 mL). To this was added pentane (3.5 mL), followed by stirring. The precipitated crystals were collected by filtration to give the title compound (0.28 g) as a colorless powder.

Anal. calcd for $C_{11}H_{18}FN_3O \cdot HBr$: C,42.87;H,6.21;N,13.63;Br, 25.93;F,6.16. Found:C,42.98;H,6.26;N,13.54;Br, 25.85;F,6.15.

Example 3

Synthesis of (2S,4R)-1-[(2S,3S)-2-amino-3-methylpentanoyl]-2-cyano-4-fluoropyrrolidine hydrochloride

(1) Synthesis of (2S,4R)-2-(aminocarbonyl)-1-(tert-butoxycarbonyl)-4-fluoropyrrolidine

According to the manner similar to that of Example 1(1), the title compound (270 mg) was obtained as a colorless gummy substance from (2S,4R)-1-(tert-butoxycarbonyl)-4-fluoropyrrolidine-2-carboxylic acid (370 mg) obtained in Reference Example 2.

MS(ESI pos.)m/z: 255($[M+Na]^+$), (ESI neg.)m/z: 231($[M-H]^-$)

(2) Synthesis of (25,4R)-2-(aminocarbonyl)-1-[(2S,3S)-2-(fluorenylmethoxycarbonylamino)-3-methylpentanoyl]-4-fluoropyrrolidine

In 4M hydrochloric acid - dioxane (3 ml) was suspended (2S,4R)-2-(aminocarbonyl)-1-(tert-butoxycarbonyl)-4-fluoropyrrolidine (260 mg) and, after stirring at room temperature for 2 hours, the reaction solution was concentrated under reduced pressure. To the residue was added chloroform (10 ml), followed by further concentration under reduced pressure. The procedure was repeated 3 times. The resulting residue, (2S,3S)-2-(fluorenylmethoxycarbonylamino)-3-methylpentanoic acid (400 mg) was dissolved in N,N-dimethylformamide (5 mL), and then 1-hydroxybenzotriazole monohydrate (210 mg), 1-(3,3-dimethylaminopropyl)-3-ethylcarbodiimide hydrochloride (260 mg) and diisopropylethylamine (0.20 mL) were added with ice-cooling. The temperature was gradually raised, and then, the mixture was stirred at room temperature overnight. The reaction mixture was taken up in water and extracted with ethyl acetate. The organic phase was washed with 0.1 M aqueous hydrochloric acid solution and a saturated aqueous sodium chloride solution, successively, dried over anhydrous sodium sulfate and, after removal of the drying agent by filtration, concentrated under reduced pressure. The residue was purified by a silica gel column chromatography (hexane : ethyl acetate =1:1 - 1:4) to give the title compound (450 mg) as a colorless amorphous substance.

MS(ESI pos.)m/z: 490($[M+Na]^+$).

(3) Synthesis of (2S,4R)-2-cyano-1-[(2S,3S)-2-(fluorenylmethoxycarbonylamino)-3-methylpentanoyl]-4-fluoropyrrolidine

According to the manner similar to that of Example 1(4), the title compound (330 mg) was obtained as a pale yellow amorphous substance from (2S,4R)-2-(aminocarbonyl)-1-[(2S,3S)-2-(fluorenylmethoxycarbonylamino)-3-methylpentanoyl]-4-fluoropyrrolidine (440 mg).

MS(ESI pos.)m/z: 472($[M+Na]^+$).

(4) Synthesis of (2S,4R)-1-[(2S,3S)-2-amino-3-methylpentanoyl]-2-cyano-4-fluoropyrrolidine hydrochloride

According to the manner similar to that of Example 1(5), the title compound (60 mg) was obtained as a colorless powder from (2S,4R)-2-cyano-1-[(2S,3S)-2-(fluorenylmethoxycarbonylamino)-3-methylpentanoyl]-4-fluoropyrrolidine (320 mg).
MS(ESI pos.)m/z: 228($[M+H]^+$), 250($[M+Na]^+$), (ESI neg.)m/z: 262($[M+CL]^-$)

Example 4

Synthesis of (2S,4S)-2-cyano-1-[2-[(5-nitropyridin-2-yl)amino]ethylamino]acetyl-4-fluoropyrrolidine maleate

(1) Synthesis of (2S,4S)-2-(aminocarbonyl)-1-bromoacetyl-4-fluoropyrrolidine

In tetrahydrofuran (10 mL) was suspended (2S,4S)-2-aminocarbonyl-4-fluoropyrrolidine hydrochloride (650 mg) obtained in Example 1(2), and then potassium 2-ethylhexanoate (1.6 g) was added with ice-cooling, followed by stirring for an hour. Bromoacetyl bromide (0.37 mL) was added with ice-cooling, and stirring was continued with ice-cooling for 30 minutes and at room temperature for an hour. To the reaction solution was added chloroform-methanol (10:1, 50 mL), followed by stirring at room temperature for 15 minutes. The precipitated salt was separated by filtration, and the filtrate was concentrated under reduced pressure. The residue was purified by a silica gel column chromatography (developing solvent; chloroform : methanol =40:1 - 25:1) to give the title compound (570 mg) as a colorless amorphous substance.

MS(ESI pos.)m/z: 275($[M+Na]^+$), 277($[M+Na]^+$).

(2) Synthesis of (2S,4S)-1-bromoacetyl-2-cyano-4-fluoropyrrolidine

In tetrahydrofuran (6 mL) was dissolved (2S,4S)-2-(aminocarbonyl)-1-bromoacetyl-4-fluoropyrrolidine (560 mg), and then trifluoroacetic anhydride (0.62 mL) was added with ice-cooling, followed by stirring with ice-cooling for an hour. The reaction solution was concentrated under reduced pressure, and the residue was purified by a silica gel column chromatography (developing solvent; chloroform : methanol =50:1 - 30:1) to give the title compound (540 mg) as a colorless solid.

MS(ESI pos.)m/z: 257($[M+Na]^+$), 259($[M+Na]^+$).

(3) Synthesis of (2S,4S)-2-cyano-1-[2-[(5-nitropyridin-2-yl)amino]ethylamino]acetyl-4-fluoropyrrolidine maleate

In tetrahydrofuran (10 mL) was dissolved 2-(2-aminoethylamino)-5-nitropyridine (580 mg), and then a tetrahydrofuran solution (2.5 mL) of (2S,4S)-1-bromoacetyl-2-cyano-4-fluoropyrrolidine (250 mg) was added with ice-cooling. The temperature was gradually raised, and then, the mixture was stirred at room temperature overnight. The solution was concentrated under reduced pressure, and the resulting residue was purified by a silica gel column chromatography (developing solvent; chloroform : methanol =50:1 - 25:1). The resulting residue was dissolved in ethanol (1 mL), and then an ethanol solution (1 mL) of maleic acid (52 mg) was added. To the reaction solution was added diethyl ether and, after removal of the supernatant, the precipitate was washed with diethyl ether. The residue was dried under reduced pressure to give the title compound (160 mg) as a yellow powder.

MS(ESI pos.)m/z: 337($[M+H]^+$), 359($[M+Na]^+$), (ESI neg.)m/z: 335($[M-H]^-$).

Example 5

Synthesis of (2S,4S)-2-cyano-1-[2-[(5-cyanopyridin-2-yl)amino]ethylamino]acetyl-4-fluoropyrrolidine maleate

According to the manner similar to that of Example 4(3), the title compound (70 mg) was obtained as a colorless powder from 2-(2-aminoethylamino)-5-cyanopyridine (520 mg) and (2S,4S)-1-bromoacetyl-2-cyano-4-fluoropyrrolidine (250 mg).

MS(ESI pos.)m/z: 317($[M+H]^+$), 339($[M+Na]^+$), (ESI neg.)m/z: 315([M-H]-).

Example 6

Synthesis of (2S,4R)-1-[(2S,3S)-2-amino-3-methylpentanoyl]-2-cyano-4-methoxypyrrolidine hydrochloride

(1) Synthesis of methyl (2S,4R)-1-(tert-butoxycarbonyl)-4-methoxypyrrolidine-2-carboxylate

In N,N-dimethylformamide (4 mL) - dichloromethane (1 mL) were dissolved methyl (2S,4R)-1-(tert-butoxycarbonyl)-4-hydroxypyrrolidine-2-carboxylate (400 mg) and methyl iodide (0.12 mL), and then 65% sodium hydride (oily, 60 mg) was added with ice-cooling. The temperature was gradually raised to room temperature, and then, the mixture was stirred overnight. The reaction mixture was taken up in a saturated aqueous sodium bicarbonate solution and extracted with ethyl acetate. The organic phase was washed with 5% aqueous sodium thiosulfate solution and a saturated aqueous sodium chloride solution, successively, dried over anhydrous sodium sulfate and, after removal of the drying agent by filtration, concentrated under reduced pressure. The residue was purified by a silica gel column chromatography (developing solvent; hexane : ethyl acetate =8:1 - 4:1) to give the title compound (330 mg) as a colorless oily substance.

MS(ESI pos.)m/z: 282($[M+Na]^+$).

(2) Synthesis of (2S,4R)-1-(tert-butoxycarbonyl)-4-methoxypyrrolidine-2-carboxylic acid

In acetonitrile (3 mL) was dissolved methyl (2S,4R)-1-(tert-butoxycarbonyl)-4-methoxypyrrolidine-2-carboxylate (330 mg), 1M aqueous lithium hydroxide solution (1.5 mL) was added with ice-cooling, and stirring was continued with ice-cooling for 30 minutes and then at room temperature for 2 hours. Further, 1M aqueous lithium hydroxide solution (0.8 mL) was added, followed by stirring at room temperature for an hour. The reaction solution was taken up in a saturated aqueous sodium chloride solution and, after making the solution acidic with 1M aqueous hydrochloric acid solution (4 mL), extracted with ethyl acetate. The organic phase was washed with a saturated aqueous sodium chloride solution, dried over anhydrous sodium sulfate and, after removal of the drying agent by filtration, concentrated under reduced pressure to give the title compound as a colorless gummy substance. This intermediate was used for the next reaction without purification.

(3) Synthesis of (2S,4R)-2-(aminocarbonyl)-1-(tert-butoxycarbonyl)-4-methoxypyrrolidine

According to the manner similar to that of Example 1(1), the title compound (260 mg) was obtained as a colorless gummy substance from (2S,4R)-1-(tert-butoxycarbonyl)-4-methoxypyrrolidine-2-carboxylic acid (330 mg).

MS(ESI pos.)m/z: 267($[M+Na]^+$), (ESI neg.)m/z: 243($[M-H]^-$).

(4) Synthesis of (25,4R)-2-(aminocarbonyl)-1-[(2S,3S)-2-(fluorenylmethoxycarbonylamino)-3-methylpentanoyl]-4-methoxypyrrolidine

According the manner similar to that of Example 3(2), the title compound (400 mg) was obtained as a colorless amorphous substance from (2S,4R)-2-(aminocarbonyl)-1-(tert-butoxycarbonyl)-4-methoxypyrrolidine (250 mg).

MS(ESI pos.)m/z: 502($[M+Na]^+$).

(5) Synthesis of (2S,4R)-2-cyano-1-[(2S,3S)-2-(fluorenylmethoxycarbonylamino)-3-methylpentanoyl]-4-methoxypyrrolidine

According to the manner similar to that of Example 1(4), the title compound (260 mg) was obtained as a pale yellow amorphous substance from (2S,4R)-2-(aminocarbonyl)-1-[(2S,3S)-2-(fluorenylmethoxycarbonylamino)-3-methylpentanoyl]-4-methoxypyrrolidine (390 mg).

MS(ESI pos.)m/z: 484($[M+Na]^+$).

(6) Synthesis of (2S,4R)-1-[(2S,3S)-2-amino-3-methylpentanoyl]-2-cyano-4-methoxypyrrolidine hydrochloride

According to the manner similar to that of Example 1(5), the title compound (70 mg) was obtained as a colorless powder from (2S,4R)-2-cyano-1-[(2S,3S)-2-(fluorenylmethoxycarbonylamino)-3-methylpentanoyl]-4-methoxypyrrolidine (250 mg).

MS(ESI pos.)m/z: 240($[M+H]^+$), 262($[M+Na]^+$), (ESI neg.)m/z: 274($[M+CL]^-$).

Example 7

Synthesis of (2S,4S)-1-[(2S,3S)-2-amino-3-methylpentanoyl]-2-cyano-4-methoxypyrrolidine hydrochloride

(1) Synthesis of methyl (2S,4S)-1-(tert-butoxycarbonyl)-4-methoxypyrrolidine-2-carboxylate

According to the manner similar to that of Example 6(1), the title compound (360 mg) was obtained as a colorless oily substance from methyl (2S,4S)-1-(tert-butoxycarbonyl)-4-hydroxypyrrolidine-2-carboxylate (400 mg).
MS(ESI pos.)m/z: 282($[M+Na]^+$).

(2) Synthesis of (2S,4S)-1-(tert-butoxycarbonyl)-4-methoxypyrrolidine-2-carboxylic acid

According to the manner similar to that of Example 6(2), the title compound (310 mg) was obtained as a colorless solid from methyl (2S,4S)-1-(tert-butoxycarbonyl)-4-methoxypyrrolidine-2-carboxylate (350 mg). This intermediate was used for the next reaction without purification.

(3) Synthesis of (2S,4S)-2-(aminocarbonyl)-1-(tert-butoxycarbonyl)-4-methoxypyrrolidine

According to the manner similar to that of Example 1(1), the title compound (290 mg) was obtained as a colorless gummy substance from (2S,4S)-1-(tert-butoxycarbonyl)-4-methoxypyrrolidine-2-carboxylic acid (310 mg).
MS(ESI pos.)m/z: 267($[M+Na]^+$), (ESI neg.)m/z: 243($[M-H]^-$).

(4) Synthesis of (2S,4S)-2-(aminocarbonyl)-1-[(2S,3S)-2-(fluorenylmethoxycarbonylamino)-3-methylpentanoyl]-4-methoxypyrrolidine

According to the manner similar to that of Example 3(2), the title compound (450 mg) was obtained as a colorless amorphous substance from (2S,4S)-2-(aminocarbonyl)-1-(tert-butoxycarbonyl)-4-methoxypyrrolidine (280 mg).
MS(ESI pos.)m/z: 502($[M+Na]^+$).

(5) Synthesis of (2S,4S)-2-cyano-1-[(2S,3S)-2-(fluorenylmethoxycarbonylamino)-3-methylpentanoyl]-4-methoxypyrrolidine

According to the manner similar to that of Example 1(4), the title compound (330 mg) was obtained as a pale yellow amorphous substance from (2S,4S)-2-(aminocarbonyl)-1-[(2S,3S)-2-(fluorenylmethoxycarbonylamino)-3-methylpentanoyl]-4-methoxypyrrolidine (440 mg).
MS(ESI pos.)m/z: 484($[M+Na]^+$).

(6) Synthesis of (2S,4S)-1-[(2S,3S)-2-amino-3-methylpentanoyl]-2-cyano-4-methoxypyrrolidine hydrochloride

According to the manner similar to that of Example 1(5), the title compound (150 mg) was obtained as a colorless powder from (2S,4S)-2-cyano-1-[(2S,3S)-2-(fluorenylmethoxycarbonylamino)-3-methylpentanoyl]-4-methoxypyrrolidine (320 mg).
MS(ESI pos.)m/z: 240($[M+H]^+$), 262($[M+Na]^+$), (ESI neg.)m/z: 274($[M+CL]^-$).

Example 8

Synthesis of (2S,4R)-1-[(2S,3S)-2-amino-3-methylpentanoyl]-2-cyano-4-hydroxypyrrolidine hydrochloride

(1) Synthesis of methyl (2S,4R)-2-(aminocarbonyl)-1-[(2S,3S)-2-(fluorenylmethoxycarbonylamino)-3-methylpentanoyl]-4-hydroxypyrrolidine

According to the manner similar to that of Example 3(2), the title compound (1.30 g) was obtained as a colorless amorphous substance from (2S,4R)-1-(tert-butoxycarbonyl)-2-(aminocarbonyl)-4-hydroxypyrrolidine (0.96 g).
MS(ESI pos.)m/z: 488($[M+Na]^+$).

(2) Synthesis of (2S,4R)-2-cyano-1-[(2S,3S)-2-(fluorenylmethoxycarbonylamino)-3-methylpentanoyl]-4-hydroxypyrrolidine

In tetrahydrofuran (30 mL) was dissolved (2S,4R)-2-(aminocarbonyl)-1-[(2S,3S)-2-(fluorenylmethoxycarbonylamino)-3-methylpentanoyl]-4-hydroxypyrrolidine (1.06 g), and then trifluoroacetic anhydride (0.72 mL) was added with ice-cooling. After stirring at the same temperature for an hour, the reaction solution was concentrated under reduced pressure. To the resulting residue was added methanol (10 mL), and concentrated under reduced pressure. This procedure was repeated once more, then methanol (10 mL) was added, followed by stirring overnight. The reaction solution was concentrated under reduced pressure, and the resulting residue was purified by a silica gel column chromatography (developing solvent; hexane : ethyl acetate =1:3) to give the title compound (0.69 g) as a colorless amorphous substance.

MS(ESI pos.)m/z: 470([M+Na]$^+$).

(3) Synthesis of (2S,4R)-1-[(2S,3S)-2-amino-3-methylpentanoyl]-2-cyano-4-hydroxypyrrolidine hydrochloride

According to the manner similar to that of Example 1(5), the title compound (41 mg) was obtained as a colorless powder from (2S,4R)-2-cyano-1-[(2S,3S)-2-(fluorenylmethoxycarbonylamino)-3-methylpentanoyl]-4-hydroxypyrrolidine (151 mg).

MS(ESI pos.)m/z: 248([M+Na]$^+$), (ESI neg.)m/z: 260([M+CL]$^-$ ).

Example 9

Synthesis of (2S,4S)-1-[(2S,3S)-2-amino-3-methylpentanoyl]-2-cyano-4-hydroxypyrrolidine hydrochloride

(1) Synthesis of (2S,4S)-4-acetoxy-2-cyano-1-[(2S,3S)-2-(fluorenylmethoxycarbonylamino)-3-methylpentanoyl] pyrrolidine

In tetrahydrofuran (5 mL) were dissolved (2S,4R)-2-cyano-1-[(2S,3S)-2-(fluorenylmethoxycarbonylamino)-3-methylpentanoyl]-4-hydroxypyrrolidine (200 mg) obtained in Example 8(2) and triphenylphosphine (258 mg), and then acetic acid (0.05 mL) and diethyl azodicarboxylate (40% toluene solution, 0.47 mL) were added with ice-cooling. The temperature was raised to room temperature, and then, the mixture was stirred overnight. The reaction solution was concentrated under reduced pressure, and the resulting residue was purified by a silica gel column chromatography (developing solvent; hexane : ethyl acetate =3:2 - 1:1) to give the title compound (135 mg) as a colorless amorphous substance.

MS(ESI pos.)m/z: 512([M+Na]$^+$).

(2) Synthesis of (2S,4S)-1-[(2S,3S)-2-amino-3-methylpentanoyl]-2-cyano-4-hydroxypyrrolidine hydrochloride

In methanol (1.6 mL) was dissolved (2S,4S)-4-acetoxy-2-cyano-1-[(2S,3S)-2-(fluorenylmethoxycarbonylamino)-3-methylpentanoyl]pyrrolidine (115 mg), and then diethylamine (0.4 mL) was added at room temperature, followed by stirring at the same temperature for 9 hours. According to the purification procedure similar to that of Example 1(5), the title compound (28 mg) was obtained as a colorless amorphous substance.

MS(ESI pos.)m/z: 226([M+H]$^+$), 248([M+Na]$^+$), (ESI neg.)m/z: 260([M+CL]$^-$).

Example 10

Synthesis of (2S,4S)-1-[(2S,3S)-2-amino-3-methylpentanoyl]-4-chloro-2-cyanopyrrolidine hydrochloride

(1) Synthesis of (2S,4S)-4-chloro-2-cyano-1-[(2S,3S)-2-(fluorenylmethoxycarbonylamino)-3-methylpentanoyl] pyrrolidine

In dichloromethane (2 mL) - carbon tetrachloride (2 mL) was dissolved (2S,4R)-2-cyano-1-[(2S,3S)-2-(fluorenylmethoxycarbonylamino)-3-methylpentanoyl]-4-hydroxypyrrolidine (200 mg) obtained in Example 8 (2), and then triphenylphosphine (234 mg) was added, followed by stirring at room temperature overnight. To the reaction solution was added ethanol (0.5 mL), followed by stirring at room temperature for 4 hours. The reaction solution was concentrated under reduced pressure, and the resulting residue was purified by a silica gel column chromatography (developing solvent; hexane : ethyl acetate =1:1 - 2:3) to give the title compound (126 mg) as a colorless amorphous substance.

MS(ESI pos.)m/z: 488([M+Na]$^+$).

(2) Synthesis of (2S,4S)-1-[(2S,3S)-2-amino-3-methylpentanoyl]-4-chloro-2-cyanopyrrolidine hydrochloride

According to the manner similar to that of Example 1 (5), the title compound (32 mg) was obtained as a colorless powder from (2S,4S)-4-chloro-2-cyano-1-[(2S,3S)-2-(fluorenylmethoxycarbonylamino)-3-methylpentanoyl] pyrrolidine (100 mg).
MS(ESI pos.)m/z: 266([M+Na]$^+$).

Example 11

Synthesis of (2S)-1-[(2S,3S)-2-amino-3-methylpentanoyl]-2-cyano-4-oxopyrrolidine trifluoroacetate

(1) Synthesis of (2S,4R)-2-(aminocarbonyl)-1-[(2S,3S)-2-(tert-butoxycarbonylamino)-3-methylpentanoyl]-4-hydroxypyrrolidine

According to the manner similar to that of Example 3 (2), the title compound (260 mg) was obtained as a colorless amorphous substance from (2S,4R)-2-(aminocarbonyl)-1-(tert-butoxycarbonyl)-4-hydroxypyrrolidine (276 mg) obtained in Reference Example 3 and (2S,3S)-2-(tert-butoxycarbonylamino)-3-methylpentanoic acid (290 mg).
MS(ESI pos.)m/z: 366([M+Na]+), (ESI neg.)m/z: 342([M-H]$^-$).

(2) Synthesis of (2S)-2-(aminocarbonyl)-1-[(2S,3S)-2-(tert-butoxycarbonylamino)-3-methylpentanoyl]-4-oxopyrrolidine

In dichloromethane (10 mL) was dissolved (2S,4R)-2-(aminocarbonyl)-1-[(2S,3S)-2-(tert-butoxycarbonylamino)-3-methylpentanoyl]-4-hydroxypyrrolidine (250 mg), and then Molecular Sieves-4A (1.5 g), pyridinium chlorochromate (235 mg) and acetic acid (0.07 mL) were added, followed by stirring at room temperature for 2 hours. The reaction solution was directly purified by a silica gel column chromatography (developing solvent; hexane : ethyl acetate =1:1 - 1:3) to give the title compound (180 mg) as a brown amorphous substance.
MS(ESI pos.)m/z: 364([M+Na]$^+$), (ESI neg.)m/z: 340([M-H]$^-$).

(3) Synthesis of (2S)-1-[(2S,3S)-2-(tert-butoxycarbonylamino)-3-methylpentanoyl]-2-cyano-4-oxopyrrolidine

In tetrahydrofuran (10 mL) was dissolved (2S)-2-(aminocarbonyl)-1-[(2S,3S)-2-(tert-butoxycarbonylamino)-3-methylpentanoyl]-4-oxopyrrolidine (168 mg) and, after ice-cooling, trifluoroacetic anhydride (0.21 mL) and diisopropylethylamine (0.51 mL) were added, followed by stirring with ice-cooling for an hour. The reaction solution was diluted with ethyl acetate (100 mL), and washed with water, 10% aqueous potassium bisulfate solution, water, a saturated aqueous sodium bicarbonate solution and a saturated aqueous sodium chloride solution, successively, and the organic phase was dried over anhydrous magnesium sulfate. After removal of the drying agent by filtration, the solvent was evaporated under reduced pressure to give the title compound (174 mg) as a brown amorphous substance.
MS(ESI pos.)m/z: 346([M+Na]$^+$), (ESI neg.)m/z: 322([M-H]$^-$).

(4) Synthesis of (2S)-1-[(2S,3S)-2-amino-3-methylpentanoyl]-2-cyano-4-oxopyrrolidine trifluoroacetate

In ice-cooled trifluoroacetic acid (0.5 mL) was dissolved (2S)-1-[(2S,3S)-2-(tert-butoxycarbonylamino)-3-methylpentanoyl]-2-cyano-4-oxopyrrolidine (91 mg) and, after stirring at room temperature for 20 minutes, the solvent was evaporated under reduced pressure. To the residue was added diisopropyl ether (10 mL), and the supernatant was removed. To the residue was again added diisopropyl ether (10 mL), and an insoluble substance was collected by filtration to give the title compound (76 mg) as a brown solid.
MS(ESI pos.)m/z: 246([M+Na]$^+$).

Example 12

Synthesis of (2S,3S)-1-[(2S,3S)-2-amino-3-methylpentanoyl]-2-cyano-3-hydroxypyrrolidine

(1) Synthesis of (2S,3S)-2-(aminocarbonyl)-1-(tert-butoxycarbonyl)-3-hydroxypyrrolidine

According to the manner similar to that of Reference Example 3, the title compound (3.19 g) was obtained

as a colorless solid from (2S,3S)-1-(tert-butoxycarbonyl)-3-hydroxypyrrolidine-2-carboxylic acid (3.47 g).
MS(ESI pos.)m/z: 253([M+Na]$^+$), (ESI neg.)m/z: 229([M-H]$^-$).

(2) Synthesis of (2S,3S)-2-(aminocarbonyl)-3-hydroxypyrrolidine hydrochloride

According to the manner similar to that of Example 1(2), the title compound was obtained from (2S,3S)-2-(aminocarbonyl)-1-(tert-butoxycarbonyl)-3-hydroxypyrrolidine (1.11 g). This intermediate was used for the next reaction without purification.

(3) Synthesis of (2S,3S)-2-(aminocarbonyl)-1-[(2S,3S)-2-(fluorenylmethoxycarbonylamino)-3-methylpentanoyl]-3-hydroxypyrrolidine

In dichloromethane (20 mL) was suspended (2S,3S)-2-(fluorenylmethoxycarbonylamino)-3-methylpentanoic acid (1.87 g), and then thionyl chloride (3.81 mL) was added, followed by reflux with heating for 20 minutes. The solvent was evaporated under reduced pressure, toluene (20 mL) was added to the residue, and the solvent was evaporated under reduced pressure. This procedure was repeated once more to give the crude product of the acid chloride. In N, N-dimethylformamide (20 mL) was dissolved (2S,3S)-2-(aminocarbonyl)-3-hydroxypyrrolidine hydrochloride obtained in the above (2), and diisopropylethylamine (1.00 mL) was added, followed by ice-cooling. To this solution was added dropwise a N,N-dimethylformamide solution (10 mL) of the previously resulting acid chloride, followed by stirring with ice-cooling for 20 minutes. The reaction solution was diluted with ethyl acetate (100 mL) and washed with a saturated aqueous sodium chloride solution, a saturated aqueous sodium bicarbonate solution and a saturated aqueous sodium chloride solution, successively, and the organic phase was dried over anhydrous magnesium sulfate. After removal of the drying agent by filtration, the solvent was evaporated under reduced pressure, and the resulting residue was purified by a silica gel column chromatography (developing solvent; chloroform : methanol =100:2 - 100:5) to give the title compound (1.08 g) as a colorless amorphous substance.
MS(ESI pos.)m/z: 488([M+Na]$^+$).

(4) Synthesis of (2S,3S)-2-cyano-1-[(2S,3S)-2-(fluorenylmethoxycarbonylamino)-3-methylpentanoyl]-3-hydroxypyrrolidine

According to the manner similar to that of Example 8(2), the title compound (795 mg) was obtained as a colorless amorphous substance from (2S,3S)-2-(aminocarbonyl)-1-[(2S,3S)-2-(fluorenylmethoxycarbonylamino)-3-methylpentanoyl]-3-hydroxypyrrolidine (920 mg).
MS(ESI pos.)m/z: 470([M+Na]$^+$).

(5) Synthesis of (2S,3S)-1-[(2S,3S)-2-amino-3-methylpentanoyl]-2-cyano-3-hydroxypyrrolidine

In methanol (1.8 mL) was dissolved (2S,3S)-2-cyano-1-[(2S,3S)-2-(fluorenylmethoxycarbonylamino)-3-methylpentanoyl]-3-hydroxypyrrolidine (415 mg), and then diethylamine (0.4 mL) was added, followed by stirring at room temperature for 4 hours. The solution was concentrated under reduced pressure, and the residue was purified by a silica gel column chromatography (developing solvent; chloroform : methanol : 25% aqueous ammonia solution =40:1:0.1 - 25:1:0.1) to give the title compound (185 mg) as a colorless oily substance.
MS(ESI pos.)m/z: 248([M+Na]$^+$).

Example 13

Synthesis of (2S,3R)-1-[(2S,3S)-2-amino-3-methylpentanoyl]-2-cyano-3-fluoropyrrolidine trifluoroacetate

(1) Synthesis of (2S,3S)-1-[(2S,3S)-2-(tert-butoxycarbonylamino)-3-methylpentanoyl]-2-cyano-3-hydroxypyrrolidine

In dichloromethane (5 mL) was dissolved (2S,3S)-1-[(2S,3S)-2-amino-3-methylpentanoyl]-2-cyano-3-hydroxypyrrolidine (170 mg) obtained in Example 12 (5), and then di-tert-butyl dicarboxylate (198 mg) and diisopropylethylamine (0.158 mL) were added with ice-cooling, followed by allowing to stand at 5°C for 2 days. The solution was concentrated under reduced pressure, and the residue was purified by a silica gel column chromatography (developing solvent; hexane : ethyl acetate =1:1) to give the title compound (173 mg) as a colorless amorphous substance.
MS(ESI pos.)m/z: 348([M+Na]$^+$), (ESI neg.)m/z: 324([M-H]$^-$).

(2) Synthesis of (2S,3R)-1-[(2S,3S)-2-(tert-butoxycarbonylamino)-3-methylpentanoyl]-2-cyano-3-fluoropyrrolidine

According to the manner similar to that of Example 54, the title compound (65 mg) was obtained as a colorless amorphous substance from (2S,3S)-1-[(2S,3S)-2-(tert-butoxycarbonylamino)-3-methylpentanoyl]-2-cyano-3-hydroxypyrrolidine (168 mg).

MS(ESI pos.)m/z. 350([M+Na]$^+$).

(3) Synthesis of (2S,3R)-1-[(2S,3S)-2-amino-3-methylpentanoyl]-2-cyano-3-fluoropyrrolidine trifluoroacetate

According to the manner similar to that of Example 11(4), the title compound (32 mg) was obtained as a yellow powder from (2S,3R)-1-[(2S,3S)-2-(tert-butoxycarbonylamino)-3-methylpentanoyl]-2-cyano-3-fluoropyrrolidine (62 mg).

MS(ESI pos.)m/z: 250([M+Na]$^+$).

Example 14

Synthesis of (2S)-1-[(2S,3S)-2-amino-3-methylpentanoyl]-2-cyano-4,4-difluoropyrrolidine hydrochloride

(1) Synthesis of (2S)-2-(aminocarbonyl)-1-(tert-butoxycarbonyl)-4,4-difluoropyrrolidine

According to the manner similar to that of Example 1(1), the title compound (2.8 g) was obtained as a colorless powder from (2S)-1-(tert-butoxycarbonyl)-4,4-difluoropyrrolidine-2-carboxylic acid (3.2 g).

MS(ESI pos.)m/z: 273([M+Na]$^+$), (ESI neg.)m/z: 249([M-H]-).

(2) Synthesis of (2S)-2-(aminocarbonyl)-4,4-difluoropyrrolidine hydrochloride

According to the manner similar to that of Example 1(2), the title compound (3.9 g) was obtained as a colorless powder from (2S)-2-(aminocarbonyl)-1-(tert-butoxycarbonyl)-4,4-difluoropyrrolidine (5.4 g).

(ESI neg.)m/z: 149([M-H]$^-$), 185([M+CL]$^-$).

(3) Synthesis of (2S)-2-(aminocarbonyl)-1-[(2S,3S)-2-(tert-butoxycarbonylamino)-3-methylpentanoyl]-4,4-difluoropyrrolidine

According to the manner similar to that of Example 1(3), the title compound (1.0 g) was obtained as a colorless amorphous substance from (2S)-2-(aminocarbonyl)-4,4-difluoropyrrolidine hydrochloride (0.56 g) and (2S,3S)-2-(tert-butoxycarbonylamino)-3-methylpentanoic acid (0.70 g).

MS(ESI pos.)m/z: 386([M+Na]$^+$), (ESI neg.)m/z: 362([M-H]$^-$).

(4) Synthesis of (2S)-1-[(2S,3S)-2-(tert-butoxycarbonylamino)-3-methylpentanoyl]-2-cyano-4,4-difluoropyrrolidine

In N,N-dimethylformamide (2.5 mL) was dissolved (2S)-2-(aminocarbonyl)-1-[(2S,3S)-2-(tert-butoxycarbonylamino)-3-methylpentanoyl]-4,4-difluoropyrrolidine (0.90 g), and then cyanuric chloride (0.28 g) was added, followed by stirring at room temperature for an hour. The reaction solution was taken up in water, and extracted with ethyl acetate. The organic phase was washed with a saturated aqueous sodium bicarbonate solution and a saturated aqueous sodium chloride solution, successively, and dried over anhydrous sodium sulfate. The drying agent was removed by filtration, and the filtrate was concentrated under reduced pressure. The residue was purified by a silica gel column chromatography (developing solvent; hexane : ethyl acetate =20:1 - 4:1) to give the title compound (0.76 g) as a colorless amorphous substance.

MS(ESI pos.)m/z: 368([M+Na]$^+$), (ESI neg.)m/z: 344([M-H]$^-$).

(5) Synthesis of (2S)-1-[(2S,3S)-2-amino-3-methylpentanoyl]-2-cyano-4,4-difluoropyrrolidine hydrochloride

To (2S)-1-[(2S,3S)-2-(tert-butoxycarbonylamino)-3-methylpentanoyl]-2-cyano-4,4-difluoropyrrolidine (0.56 g) was added 2M aqueous hydrochloric acid solution (12 mL), followed by stirring at room temperature overnight. To the solution was added an additional 2M aqueous hydrochloric acid solution (6 mL) and, after stirring at room temperature overnight, the aqueous solution was washed with ethyl acetate. To the aqueous phase were added 1M aqueous sodium hydroxide solution (35 mL) and an excess amount of sodium chloride and, after stirring, the mixture was taken up in a saturated aqueous sodium bicarbonate solution and extracted with ethyl acetate. The organic phase was washed

with a saturated aqueous sodium chloride solution and dried over anhydrous sodium sulfate. After removal of the drying agent by filtration, the filtrate was concentrated under reduced pressure to give (2S)-1-[(2S,3S)-2-amino-3-methylpentanoyl]-2-cyano-4,4-difluoropyrrolidine, which was then dissolved in diethyl ether (20 mL), followed by addition of 4M hydrochloric acid (ethyl acetate solution, 0.50 mL) with ice-cooling. The precipitated insoluble substance was collected by filtration to give the title compound (0.37 g) as a colorless powder.

MS(ESI pos.)m/z: 246($[M+H]^+$), 268($[M+Na]^+$), (ESI neg.)m/z: 244([M-H]-), 280($[M+CL]^-$).

Example 15

Synthesis of (2S,4S)-1-[(2S)-2-amino-3-methylbutanoyl]-2-cyano-4-fluoropyrrolidine hydrochloride

(1) Synthesis of (2S,4S)-2-(aminocarbonyl)-1-[(2S)-2-(tert-butoxycarbonylamino)-3-methylbutanoyl]-4-fluoropyrrolidine

According to the manner similar to that of Example 1(3), the title compound (4.22 g) was obtained as a colorless amorphous substance from (2S,4S)-2-(aminocarbonyl)-4-fluoropyrrolidine hydrochloride (2.33 g) and (2S)-2-(tert-butoxycarbonylamino)-3-methylbutanoic acid (3.00 g).

MS(ESI pos.)m/z: 354($[M+Na]^+$), (ESI neg.)m/z: 330($[M-H]^-$).

(2) Synthesis of (2S,4S)-1-[(2S)-2-(tert-butoxycarbonylamino)-3-methylbutanoyl]-2-cyano-4-fluoropyrrolidine

In N,N-dimethylformamide (16 mL) was dissolved (2S,4S)-2-(aminocarbonyl)-1-[(2S)-2-(tert-butoxycarbonylamino)-3-methylbutanoyl]-4-fluoropyrrolidine (4.03 g), and cyanuric chloride (1.35 g) was added, followed by stirring at room temperature for an hour. The reaction solution was taken up in water and extracted with ethyl acetate. The organic phase was washed with a saturated aqueous sodium chloride solution and dried over anhydrous sodium sulfate. After removal of the drying agent by filtration, the filtrate was concentrated under reduced pressure to give the title compound (3.49 g) as a colorless solid.

MS(ESI pos.)m/z: 336($[M+Na]^+$), (ESI neg.)m/z: 312($[M-H]^-$).

(3) Synthesis of (2S,4S)-1-[(2S)-2-amino-3-methylbutanoyl]-2-cyano-4-fluoropyrrolidine hydrochloride

In methanol (11 mL) was dissolved (2S,4S)-1-[(2S)-2-(tert-butoxycarbonylamino)-3-methylbutanoyl]-2-cyano-4-fluoropyrrolidine (1.70 g) and, after ice-cooling, 4M aqueous hydrochloric acid solution (11 mL) was added. The temperature was raised to a room temperature, and then, the mixture was stirred overnight. The methanol was concentrated under reduced pressure, and the resulting aqueous solution was washed with ethyl acetate. To the aqueous phase were added 4M aqueous sodium hydroxide solution (12 mL) and sodium chloride and, after extraction with ethyl acetate, the organic phase was washed with a saturated aqueous sodium chloride solution. The resulting organic phase was dried over anhydrous sodium sulfate, the drying agent was removed by filtration, and the filtrate was concentrated under reduced pressure to give (2S,4S)-1-[(2S)-2-amino-3-methylbutanoyl]-2-cyano-4-fluoropyrrolidine (0.80 g) as a colorless solid, an aliquot (0.60 g) of which was then dissolved in methanol, and added to a diisopropyl ether solution (22 mL) of 4M hydrochloric acid (ethyl acetate solution, 0.77 mL) with ice-cooling. The solution was stirred at room temperature for an hour, and the precipitated insoluble substance was collected by filtration to give the title compound (0.75 g) as a colorless powder.

MS(ESI pos.)m/z: 214($[M+H]^+$), 236($[M+Na]^+$), (ESI neg.)m/z: 248($[M+CL]^-$).

$^1$H-NMR(DMSO-$d_6$,500MHz)δ8.57(3H,br s),5.55(1H,br d,J=51.8Hz),5.06(1H,d,J=9.2Hz),4.08-3.90(2H,m),3.83 (1H,d,J=7.3Hz),2.55-2.34(2H,m),2.12(1H,m),1.01(3H,d,J=6.7Hz),0.98(3H,d,J=6.7Hz)

Example 16

Synthesis of (2S,4S)-1-[(2S,3R)-2-amino-3-methoxybutanoyl]-2-cyano-4-fluoropyrrolidine hydrochloride

(1) Synthesis of (2S,4S)-2-(aminocarbonyl)-1-[(2S,3R)-2-(tert-butoxycarbonylamino)-3-methoxybutanoyl)-4-fluoropyrrolidine

According to the manner similar to that of Example 1(3), the title compound (2.28 g) was obtained as a colorless amorphous substance from (2S,4S)-2-(aminocarbonyl)-4-fluoropyrrolidine hydrochloride (1.18 g) and (2S,3R)-2-(tert-butoxycarbonylamino)-3-methoxybutanoic acid (1.63 g).

MS(ESI pos.)m/z: 370($[M+Na]^+$), (ESI neg.)m/z: 346($[M-H]^-$).

(2) Synthesis of (2S,4S)-1-[(2S,3R)-2-(tert-butoxycarbonylamino)-3-methoxybutanoyl]-2-cyano-4-fluoropyrrolidine

According to the manner similar to that of Example 15(2), the title compound (1.96 g) was obtained as a colorless solid from (2S,4S)-2-(aminocarbonyl)-1-[(2S,3R)-2-(tert-butoxycarbonylamino)-3-methoxybutanoyl]-4-fluoropyrrolidine (2.17 g).

MS(ESI pos.)m/z: 352([M+Na]$^+$), (ESI neg.)m/z: 328([M-H]$^-$).

(3) Synthesis of (2S,4S)-1-[(2S,3R)-2-amino-3-methoxybutanoyl]-2-cyano-4-fluoropyrrolidine hydrochloride

According to the manner similar to that of Example 15(3), the title compound (0.61 g) was obtained as a colorless powder from (2S,4S)-1-[(2S,3R)-2-(tert-butoxycarbonylamino)-3-methoxybutanoyl]-2-cyano-4-fluoropyrrolidine (1.82 g).

MS(ESI pos.)m/z: 230([M+H]$^+$), 252[M+Na]$^+$), (ESI neg.)m/z: 264[M+CL]$^-$).

Example 17

Synthesis of (2S,4S)-1-[(2S)-2-amino-3,3-dimethylbutanoyl]-2-cyano-4-fluoropyrrolidine hydrochloride

(1) Synthesis of (2S,4S)-2-(aminocarbonyl)-1-[(2S)-2-(tert-butoxycarbonylamino)-3,3-dimethylbutanoyl]-4-fluoropyrrolidine

According to the manner similar to that of Example 1(3), the title compound (4.14 g) was obtained as a colorless amorphous substance from (2S,4S)-2-(aminocarbonyl)-4-fluoropyrrolidine hydrochloride (2.00 g) and (2S)-2-(tert-butoxycarbonylamino)-3,3-dimethylbutanoic acid (2.74 g).

MS(ESI pos.)m/z: 368([M+Na]$^+$), (ESI neg.)m/z: 344([M-H]$^-$).

(2) Synthesis of (2S,4S)-1-[(2S)-2-(tert-butoxycarbonylamino)-3,3-dimethylbutanoyl]-2-cyano-4-fluoropyrrolidine

According to the manner similar to that of Example 15(2), the title compound (2.90 g) was obtained as a colorless solid from (2S,4S)-2-(aminocarbonyl)-1-[(2S)-2-(tert-butoxycarbonylamino)-3,3-dimethylbutanoyl]-4-fluoropyrrolidine (4.10 g).

MS(ESI pos.)m/z: 350([M+Na]$^+$), (ESI neg.)m/z: 326([M-H]$^-$).

(3) Synthesis of (2S,4S)-1-[(2S)-2-amino-3,3-dimethylbutanoyl]-2-cyano-4-fluoropyrrolidine hydrochloride

According to the manner similar to that of Example 15(3), (2S,4S)-1-[(2S)-2-amino-3,3-dimethylbutanoyl]-2-cyano-4-fluoropyrrolidine (2.25 g) was obtained from (2S,4S)-1-[(2S)-2-(tert-butoxycarbonylamino)-3,3-dimethylbutanoyl]-2-cyano-4-fluoropyrrolidine (3.71 g). From an aliquot (0.80 g) of the resulting product was then obtained the title compound (0.92 g).

MS(ESI pos.)m/z: 250([M+Na]$^+$), (ESI neg.)m/z: 262([M+CL]$^-$ ).

$^1$H-NMR(DMSO-$d_6$,500MHz)δ8.54(3H,br s),5.55(1H,br d,J=51.5Hz), 5.07(1H,d,J=9.8Hz),4.15-3.93(2H,m),3.79(1H, s),2.55-2.32(2H, m),1.05(9H,s).

Example 18

Synthesis of (2S,4S)-1-[(2S)-2-amino-3,3-dimethylbutanoyl]-2-cyano-4-fluoropyrrolidine hydrobromide monohydrate

In methanol (6 mL) was dissolved (2S,4S)-1-[(2S)-2-amino-3,3-dimethylbutanoyl]-2-cyano-4-fluoropyrrolidine (0.25 g) obtained in Example 17, and then added to an ice-cooled mixture of 48% hydrobromic acid (0.14 mL) and diisopropyl ether (9 mL). Further, diisopropyl ether (5 mL) was added, followed by stirring. The precipitated crystals were collected by filtration, and the resulting crystals were dissolved in methanol (1.5 mL), added to an ice-cooled isopropyl acetate (17 mL) and stirred. The precipitated crystals were collected by filtration to give the title compound (0.20 g) as a colorless powder.

Anal.calcd. for $C_{11}H_{18}FN_3O \cdot HBr \cdot H_2O$:C,40.50;H,6.49;N,12.88;B r,24.49;F,5.82. Found:C,40.57;H,6.44;N,13.02;Br, 24.52;F,5.8 3.

Example 19

Synthesis (2S,4S)-1-[(2S,3R)-2-amino-3-methylpentanoyl]-2-cyano-4-fluoropyrrolidine hydrochloride

(1) Synthesis of (2S,4S)-2-(aminocarbonyl)-1-[(2S,3R)-2-(tert-butoxycarbonylamino)-3-methylpentanoyl]-4-fluoropyrrolidine

According to the manner similar to that of Example 1(3), the title compound (1.30 g) was obtained as a colorless amorphous substance from (2S,4S)-2-(aminocarbonyl)-4-fluoropyrrolidine hydrochloride (0.67 g) and (2S,3R)-2-(tert-butoxycarbonylamino)-3-methylpentanoic acid hemihydrate (0.96 g).
MS(ESI pos.)m/z: 368($[M+Na]^+$), (ESI neg.)m/z: 344($[M-H]^-$).

(2) Synthesis of (2S,4S)-1-[(2S,3R)-2-(tert-butoxycarbonylamino)-3-methylpentanoyl]-2-cyano-4-fluoropyrrolidine

According to the manner similar to that of Example 15(2), the title compound (1.15 g) was obtained as a colorless amorphous substance from (2S,4S)-2-(aminocarbonyl)-1-[(2S,3R)-2-(tert-butoxycarbonylamino)-3-methylpentanoyl]-4-fluoropyrrolidine (1.23 g).
MS(ESI pos.)m/z: 350($[M+Na]^+$), (ESI neg.)m/z: 326($[M-H]^-$).

(3) Synthesis of (2S,4S)-1-[(2S,3R)-2-amino-3-methylpentanoyl]-2-cyano-4-fluoropyrrolidine hydrochloride

According to the manner similar to that of Example 15(3), the title compound (0.29 g) was obtained from (2S,4S)-1-[(2S,3R)-2-(tert-butoxycarbonylamino)-3-methylpentanoyl]-2-cyano-4-fluoropyrrolidine (1.08 g).
MS(ESI pos.)m/z: 228($[M+H]^+$), 250($[M+Na]^+$), (ESI neg.)m/z: 262($[M+CL]^-$).

Example 20

Synthesis of (2S,4S)-1-[(2S,3S)-2-amino-3-methylpentanoyl]-2-cyano-4-fluoropyrrolidine hydrochloride

(1) Synthesis of (2S,4S)-2-(aminocarbonyl)-1-[(2S,3S)-2-(tert-butoxycarbonylamino)-3-methylpentanoyl]-4-fluoropyrrolidine

According to the manner similar to that of Example 1(3), the title compound (0.99 g) was obtained as a colorless amorphous substance from (2S,4S)-2-(aminocarbonyl)-4-fluoropyrrolidine hydrochloride (0.51 g) and (2S,3S)-2-(tert-butoxycarbonylamino)-3-methylpentanoic acid (0.69 g).
MS(ESI pos.)m/z: 368($[M+Na]^+$), (ESI neg.)m/z: 344($[M-H]^-$).

(2) Synthesis of (2S,4S)-1-[(2S,3S)-2-(tert-butoxycarbonylamino)-3-methylpentanoyl]-2-cyano-4-fluoropyrrolidine

According to the manner similar to that of Example 15(2), the title compound (0.83 g) was obtained as a colorless amorphous substance from (2S,4S)-2-(aminocarbonyl)-1-[(2S,3S)-2-(tert-butoxycarbonylamino)-3-methylpentanoyl]-4-fluoropyrrolidine (0.84 g).
MS(ESI pos.)m/z: 350($[M+Na]^+$), (ESI neg.)m/z: 326([M-H]-).

(3) Synthesis of (2S,4S)-1-[(2S,3S)-2-amino-3-methylpentanoyl)-2-cyano-4-fluoropyrrolidine hydrochloride

According to the manner similar to that of Example 15(3), the title compound (0.14 g), which was identical with the compound obtained in Example 1, was obtained from (2S,4S)-1-[(2S,3S)-2-(tert-butoxycarbonylamino)-3-methylpentanoyl]-2-cyano-4-fluoropyrrolidine (0.35 g).

Example 21

Synthesis of (2S,4S)-1-(tert-butylamino)acetyl-2-cyano-4-fluoropyrrolidine hydrochloride

In tetrahydrofuran (10 mL) was dissolved tert-butylamine (0.47 g), and a tetrahydrofuran solution (3 mL) of (2S,4S)-1-bromoacetyl-2-cyano-4-fluoropyrrolidine (0.30 g) was added with ice-cooling. The temperature was gradually raised and, after stirring at room temperature overnight, the solution was concentrated under reduced pressure. To the resulting residue was added a saturated aqueous sodium bicarbonate solution, followed by extraction with chloroform. The organic phase was washed with a saturated aqueous sodium chloride solution and dried over anhydrous sodium sulfate and, after removal of the drying agent by filtration, the filtrate was concentrated under reduced pressure. The resulting residue was suspended in diethyl ether, and an insoluble substance was collected by filtration to give (2S,4S)-1-(tert-butylamino)acetyl-2-cyano-4-fluoropyrrolidine (0.26 g) as a colorless powder, an aliquot (0.25 g) of which was then added to an ice-cooled diethyl ether solution of 4M hydrochloric acid (ethyl acetate solution, 0.3 mL), followed by stirring at room temperature for an hour. An insoluble substance was collected by filtration to give the title compound (0.28 g) as a colorless powder.

MS(ESI pos.)m/z: 228($[M+H]^+$), 250($[M+Na]^+$), (ESI neg.)m/z: 226($[M-H]^-$), 262($[M+CL]^-$).

$^1$H-NMR(DMSO-$d_6$,500MHz)δ9.10(2H,br s),5.56(1H,br d,J=52.9Hz), 5.09-5.06(1H,m),4.16(1H,dd,J=24.4,12.5Hz), 4.12(1H,d,J=16.5H z),3.88(1H,d,J=16.5Hz),3.86(1H,ddd,J=39.9,12.5,3.3Hz),2.54-2.40(2H,m),1.33(9H,s).

Example 22

Synthesis of (2S,4S)-2-cyano-4-fluoro-1-(isopropylamino)acetylpyrrolidine hydrochloride

According to the manner similar to that of Example 21, the title compound (0.16 g) was obtained as a colorless powder from isopropylamine (0.65 g) and (2S,4S)-1-bromoacetyl-2-cyano-4-fluoropyrrolidine (0.50 g).

MS(ESI pos.)m/z: 214($[M+H]^+$), 236($[M+Na]^+$), (ESI neg.)m/z: 212($[M-H]^-$), 248($[M+CL]^-$).

Example 23

Synthesis of (2S,4S)-2-cyano-1-(cyclopropylamino)acetyl-4-fluoropyrrolidine hydrochloride

According to the manner similar to that of Example 21, the title compound (0.28 g) was obtained as a colorless powder from cyclopropylamine (0.86 g) and (2S,4S)-1-bromoacetyl-2-cyano-4-fluoropyrrolidine (0.71 g).

MS(ESI pos.)m/z: 212($[M+H]^+$), 234($[M+Na]^+$), (ESI neg.)m/z: 246($[M+CL]^-$).

Example 24

Synthesis of (2S,4S)-2-cyano-1-(cyclobutylamino)acetyl-4-fluoropyrrolidine hydrochloride

According to the manner similar to that of Example 21, the title compound (0.31 g) was obtained as a colorless powder from cyclobutylamine (1.07 g) and (2S,4S)-1-bromoacetyl-2-cyano-4-fluoropyrrolidine (0.71 g).

MS(ESI pos.)m/z: 226($[M+H]^+$), 248($[M+Na]^+$), (ESI neg.)m/z: 260($[M+CL]^-$).

Example 25

Synthesis of (2S,4S)-2-cyano-1-(cyclopentylamino)acetyl-4-fluoropyrrolidine hydrochloride

According to the manner similar to that of Example 21, the title compound (0.58 g) was obtained as a colorless powder from cyclopentylamine (1.28 g) and (2S,4S)-1-bromoacetyl-2-cyano-4-fluoropyrrolidine (0.71 g).

MS(ESI pos.)m/z: 240($[M+H]^+$), 262($[M+Na]^+$), (ESI neg.)m/z: 274($[M+CL]^-$).

Example 26

Synthesis of (2S,4S)-2-cyano-4-fluoro-1-[(1-hydroxymethyl)cyclopentylamino]acetylpyrrolidine hydrochloride

According to the manner similar to that of Example 21, the title compound (0.51 g) was obtained as a colorless powder from (1-hydroxymethyl)cyclopentylamine (0.59 g) and (2S,4S)-1-bromoacetyl-2-cyano-4-fluoropyrrolidine (0.60 g).

MS(ESI pos.)m/z: 292($[M+Na]^+$), (ESI neg.)m/z: 268($[M-H]^-$), 304($[M+CL]^-$).

$^1$H-NMR(DMSO-$d_6$,500MHz)δ8.99(2H,br s),5.68(1H,br s),5.55(1H,b r d,J=52.4Hz),5.08-5.05(1H,m),4.17(1H,br d, J=16.5Hz),4.09(1 H,dd,J=23.1,12.2Hz),3.98(1H,br d,J=16.5Hz),3.82(1H,ddd,J=39 .3,12.2,3.1Hz),3.51&3.48(2H,ABq, J=12.5Hz),2.56-2.36(2H,m),1 .86-1.68(6H,m),1.59-1.48(2H,m).

Example 27

Synthesis of (2S,4S)-2-cyano-4-fluoro-1-(3-isopropoxypropylamino)acetylpyrrolidine hydrochloride

In tetrahydrofuran (15 mL) was dissolved 3-isopropoxypropylamine (1.1 g), and then a tetrahydrofuran solution

(5 mL) of (2S,4S)-1-bromoacetyl-2-cyano-4-fluoropyrrolidine (0.45 g) was added with ice-cooling. The temperature was gradually raised, and then, the mixture was stirred at room temperature overnight. The solution was concentrated under reduced pressure and dissolved in chloroform, and the organic phase was washed with a saturated aqueous sodium bicarbonate solution and a saturated aqueous sodium chloride solution, successively, and dried over anhydrous sodium sulfate. The drying agent was removed by filtration, and the filtrate was concentrated under reduced pressure. The residue was purified by a silica gel column chromatography (developing solvent; chloroform : methanol =50:1 - 25:1). The resulting residue was dissolved in diethyl ether (5 mL), and added to a diethyl ether solution (40 mL) of 4M hydrochloric acid (ethyl acetate solution, 0.33 mL) with ice-cooling. The precipitated insoluble substance was collected by filtration, and washed with diethyl ether to give the title compound (0.32 g) as a colorless powder.

MS(ESI pos.)m/z: 272($[M+H]^+$), 294($[M+Na]^+$), (ESI neg.)m/z: 270($[M-H]^-$), 306($[M+CL]^-$),

Example 28

Synthesis of (2S,4S)-2-cyano-1-(cyclooctylamino)acetyl-4-fluoropyrrolidine hydrochloride

According to the manner similar to that of Example 27, the title compound (0.29 g) was obtained as a colorless powder from cyclooctylamine (1.1 g) and (2S,4S)-1-bromoacetyl-2-cyano-4-fluoropyrrolidine (0.45 g).

MS(ESI pos.)m/z: 282($[M+H]^+$), 304($[M+Na]^+$), (ESI neg.)m/z: 280($[M-H]^-$), 316($[M+CL]^-$).

Example 29

Synthesis of (25,4S)-2-cyano-1-[2-(3,4-dimethoxyphenyl)ethylamino]acetyl-4-fluoropyrrolidine hydrochloride

According to the manner similar to that of Example 27, the title compound (0.24 g) was obtained as a colorless powder from 2-(3,4-dimethoxyphenyl)ethylamine (0.5 g) and (2S,4S)-1-bromoacetyl-2-cyano-4-fluoropyrrolidine (0.25 g).

MS(ESI pos.)m/z: 336($[M+H]^+$), 358($[M+Na]^+$), (ESI neg.)m/z: 334($[M-H]^-$), 370($[M+CL]^-$).

Example 30

Synthesis of (2S,4S)-2-cyano-4-fluoro-1-[(1-methoxymethyl-1-methyl)ethylamino]acetylpyrrolidine hydrochloride

In a mixture of tetrahydrofuran (7.5 mL) and ethanol (2.5 mL) was dissolved (1-methoxymethyl-1-methyl) ethylamine hydrochloride (0.74 g) and, after neutralization by adding diisopropylamine (0.92 mL) with ice-cooling, (2S, 4S)-1-bromoacetyl-2-cyano-4-fluoropyrrolidine (0.62 g) was added. After stirring with ice-cooling for an hour, the temperature was returned to room temperature, and then, the mixture was stirred for 2 days. Working up in the manner similar to that of Example 21, the title compound (0.07 g) was obtained as a colorless powder.

MS(ESI pos.)m/z: 258($[M+H]^+$), 280($[M+Na]^+$), (ESI neg.)m/z: 292($[M+CL]^-$).

Example 31

Synthesis of (2S,4S)-2-cyano-4-fluoro-1-[(2-hydroxy-1,1-dimethyl)ethylamino]acetylpyrrolidine hydrochloride

According to the manner similar to that of Example 21, the title compound (0.62 g) was obtained as a pale pink powder from 2-amino-2-methyl-1-propanol (0.71 g) and (2S,4S)-1-bromoacetyl-2-cyano-4-fluoropyrrolidine (0.54 g).

MS(ESI pos.)m/z: 244($[M+H]^+$), 266($[M+Na]^+$), (ESI neg.)m/z: 242($[M-H]^-$), 278($[M+CL]^-$).

Example 32

Synthesis of (2S,4S)-1-(2-adamantylamino)acetyl-2-cyano-4-fluoropyrrolidine hydrochloride

According to the manner similar to that of Example 27, the title compound (0.23 g) was obtained as a colorless powder from 2-adamantanamine (0.4 g) and (2S,4S)-1-bromoacetyl-2-cyano-4-fluoropyrrolidine (0.25 g).

MS(ESI pos.)m/z: 306($[M+H]^+$), 328($[M+Na]^+$), (ESI neg.)m/z: 304($[M-H]^-$), 340($[M+CL]^-$).

Example 33

Synthesis of (2S,4S)-2-cyano-4-fluoro-1-(1-hydroxy-3-adamantylamino)acetylpyrrolidine hydrochloride

According to the manner similar to that of Example 27, the title compound (0.42 g) was obtained as a colorless powder from 3-amino-1-adamantanol (0.70 g) and (2S,4S)-1-bromoacetyl-2-cyano-4-fluoropyrrolidine (0.47 g).

MS(ESI pos.)m/z: 322($[M+H]^+$), 344($[M+Na]^+$), (ESI neg.)m/z: 320($[M-H]^-$), 356($[M+CL]^-$).

Example 34

Synthesis of (2S,4S)-2-cyano-4-fluoro-1-(1-hydroxy-4-adamantylamino)acetylpyrrolidine hydrochloride

In tetrahydrofuran (10 mL) - ethanol (5 mL) was dissolved 4-amino-1-adamantanol (0.5 g), and (2S,4S)-1-bromoacetyl-2-cyano-4-fluoropyrrolidine (0.45 g) was added with ice-cooling. The temperature was gradually raised, and then, the mixture was stirred at room temperature overnight. The solution was taken up in a saturated aqueous sodium bicarbonate solution and extracted with ethyl acetate. The organic phase was washed with a saturated aqueous sodium chloride solution and dried over anhydrous sodium sulfate, the drying agent was removed by filtration, and the filtrate was concentrated under reduced pressure. The residue was purified by a silica gel column chromatography (developing solvent; chloroform : methanol : 25% aqueous ammonia solution =40:1:0.1 - 10:1:0.1). The resulting residue was dissolved in ethyl acetate (5 mL), and 4M hydrochloric acid (ethyl acetate solution, 0.30 mL) was added with ice-cooling. The precipitated insoluble substance was collected by filtration and washed with ethyl acetate to give the title compound (0.27 g) as a colorless powder.

MS(ESI pos.)m/z: 322($[M+H]^+$), 344($[M+Na]^+$), (ESI neg.)m/z: 356($[M+CL]^-$).

Example 35

Synthesis of (2S,4S)-2-cyano-4-fluoro-1-(1-methoxy-3-adamantylamino)acetylpyrrolidine hydrochloride

According to the manner similar to that of Example 34, (2S,4S)-2-cyano-4-fluoro-1-(1-methoxy-3-adamantylamino)acetylpyrrolidine (0.23 g) was obtained from 1-methoxy-3-adamantanamine (0.17 g) and (2S,4S)-1-bromoacetyl-2-cyano-4-fluoropyrrolidine (0.17 g). An aliquot (0.22 g) of this product was dissolved in ethyl acetate (4 mL), and then 4M hydrochloric acid (ethyl acetate solution, 0.20 mL) was added. To the solution was added diethyl ether (8 mL), and the precipitated insoluble substance was collected by filtration to give the title compound (0.10 g) as a colorless powder.

MS(ESI pos.)m/z: 336($[M+H]^+$), 358($[M+Na]^+$), (ESI neg.)m/z: 370($[M+CL]^-$).

Example 36

Synthesis of (2S,4S)-1-(1-adamantylamino)acetyl-2-cyano-4-fluoropyrrolidine hydrochloride

According to the manner similar to that of Example 35, the title compound (0.15 g) was obtained as a colorless powder from 1-adamantanamine (0.45 g) and (2S,4S)-1-bromoacetyl-2-cyano-4-fluoropyrrolidine (0.235 g).

MS(ESI pos.)m/z: 306($[M+H]^+$), 328($[M+Na]^+$), (ESI neg.)m/z: 340($[M+CL]^-$).

Example 37

Synthesis of (2S,4S)-1-[2-[(5-chloropyridin-2-yl)amino]ethylamino]acetyl-2-cyano-4-fluoropyrrolidine dihydrochloride

(1) Synthesis of (2S,4S)-1-[N-(tert-butoxycarbonyl)-2-[(5-chloropyridin-2-yl)amino]ethylamino]acetyl-2-cyano-4-fluoropyrrolidine

In ethanol (15 mL) was dissolved 2-(2-aminoethylamino)-5-chloropyridine (1.54 g), and then a tetrahydrofuran solution (15 mL) of (2S,4S)-1-bromoacetyl-2-cyano-4-fluoropyrrolidine (0.71 g) was added with ice-cooling, followed by stirring with ice-cooling for 10 minutes and then at room temperature for 30 minutes. The solution was again ice-cooled, and a tetrahydrofuran solution (10 mL) of di-tert-butyldicarbonate (1.96 g) and diisopropylethylamine (0.52 mL) were added. The temperature was raised, and then, the mixture was stirred followed by stirring at room temperature for an hour. The solvent was evaporated under reduced pressure, and the residue was purified by a silica gel column chromatography (developing solvent; hexane : ethyl acetate =2:1 - only ethyl acetate) to give the title compound (1.07

g) as a colorless amorphous substance.
MS(ESI pos.)m/z: 448([M+Na]$^+$), (ESI neg.)m/z: 424([M-H]$^-$)

(2) Synthesis of (2S,4S)-1-[2-[(5-chloropyridin-2-yl)amino]ethylamino]acetyl-2-cyano-4-fluoropyrrolidine dihydrochloride

In 1,4-dioxane (2.5 mL) was dissolved (2S,4S)-1-[N-(tert-butoxycarbonyl)-2-[(5-chloropyridin-2-yl)amino] ethylamino]acetyl-2-cyano-4-fluoropyrrolidine (1.02 g), and 4M hydrochloric acid (1,4-dioxane solution, 7.5 mL) was added with ice-cooling, followed by stirring with ice-cooling for an hour. To the reaction solution was added toluene (30 mL), and an insoluble substance was collected by filtration. The resulting powder was dissolved in methanol (2 mL), added to toluene (50 mL), and stirred at room temperature. The precipitated insoluble substance was collected by filtration to give the title compound (0.75 g) as a colorless powder.
MS(ESI pos.)m/z: 326([M+H]$^+$), 348([M+Na]$^+$), (ESI neg.)m/z: 324([M-H]$^-$), 360([M+CL]$^-$).

Example 38

Synthesis of (2S,4S)-2-cyano-4-fluoro-1-[2-[(pyridin-2-yl)amino]ethylamino]acetylpyrrolidine dihydrochloride

(1) Synthesis of (2S,4S)-1-[N-(tert-butoxycarbonyl)-2-[(pyridin-2-yl)amino]ethylamino]acetyl-2-cyano-4-fluoropyrrolidine

According to the manner similar to that of Example 37(1), the title compound (0.60 g) was obtained as a colorless amorphous substance from 2-(2-aminoethylamino)pyridine (0.82 g) and (2S,4S)-1-bromoacetyl-2-cyano-4-fluoropyrrolidine (0.71 g).
MS(ESI pos.)m/z: 414[[M+Na]$^+$), (ESI neg.)m/z: 390([M-H]$^-$).

(2) Synthesis of (2S,4S)-2-cyano-4-fluoro-1-[2-[(pyridin-2-yl)amino]ethylamino]acetylpyrrolidine dihydrochloride

According to the manner similar to that of Example 37(2), the title compound (0.27 g) was obtained as a colorless powder from (2S,4S)-1-[N-(tert-butoxycarbonyl)-2-[(pyridin-2-yl)amino]ethylamino]acetyl-2-cyano-4-fluoropyrrolidine (0.54 g).
MS(ESI pos.)m/z: 292([M+H]$^+$), 314([M+Na]$^+$), (ESI neg.)m/z: 290([M-H]$^-$), 326([M+CL]$^-$).

Example 39

Synthesis of (2S,4S)-1-[2-[(5-aminocarbonylpyridin-2-yl)amino]ethylamino]acetyl-2-cyano-4-fluoropyrrolidine dihydrochloride

(1) Synthesis of (2S,4S)-1-[N-(tert-butoxycarbonyl)-2-[(5-aminocarbonylpyridin-2-yl)amino]ethylamino]acetyl-2-cyano-4-fluoropyrrolidine

According to the manner similar to that of Example 37(1), the title compound (0.36 g) was obtained as a colorless amorphous substance from 2-(2-aminoethylamino)-5-aminocarbonylpyridine (1.08 g) and (2S,4S)-1-bromoacetyl-2-cyano-4-fluoropyrrolidine (0.71 g).
MS(ESI pos.)m/z: 457([M+Na]$^+$), (ESI neg.)m/z: 433([M-H]$^-$).

(2) Synthesis of (2S,4S)-1-[2-[(5-aminocarbonylpyridin-2-yl)amino]ethylamino]acetyl-2-cyano-4-fluoropyrrolidine dihydrochloride

According to the manner similar to that of Example 37(2), the title compound (0.26 g) was obtained as a pale pink powder from (2S,4S)-1-[N-(tert-butoxycarbonyl)-2-[(5-aminocarbonylpyridin-2-yl)amino]ethylamino]acetyl-2-cyano-4-fluoropyrrolidine (0.32 g).
MS(ESI pos.)m/z: 335([M+H]$^+$), 357([M+Na]$^+$), (ESI neg.)m/z: 333([M-H]$^-$), 369([M+CL]$^-$).

Example 40

Synthesis of (2S,4S)-1-[[(2S)-2-amino-2-cyclohexyl]acetyl]-2-cyano-4-fluoropyrrolidine hydrochloride

(1) Synthesis of (2S,4S)-2-(aminocarbonyl)-1-[[(2S)-2-fluorenylmethoxycarbonylamino-2-cyclohexyl]acetyl]-4-fluoropyrrolidine

According to the manner similar to that of Example 1(3), the title compound (0.88 g) was obtained as a colorless amorphous substance from (2S,4S)-2-(aminocarbonyl)-4-fluoropyrrolidine hydrochloride (0.30 g) and [(2S)-2-fluorenylmethoxycarbonylamino-2-cyclohexyl]acetic acid (0.71 g).
MS(ESI pos.)m/z: 516([M+Na]$^+$).

(2) Synthesis of (2S,4S)-2-cyano-1-[[(2S)-2-fluorenylmethoxycarbonylamino-2-cyclohexyl]acetyl]-4-fluoropyrrolidine

According to the manner similar to that of Example 1(4), the title compound (0.76 g) was obtained as a colorless amorphous substance from (2S,4S)-2-(aminocarbonyl)-1-([[(2S)-2-fluorenylmethoxycarbonylamino-2-cyclohexyl] acetyl]-4-fluoropyrrolidine (0.86 g).
MS(ESI pos.)m/z: 498([M+Na]$^+$).

(3) Synthesis of (2S,4S)-1-[[(2S)-2-amino-2-cyclohexyl]acetyl]-2-cyano-4-fluoropyrrolidine hydrochloride

According to the manner similar to that of Example 1(5), the title compound (0.27 g) was obtained as a colorless powder from (2S,4S)-2-cyano-1-[[(2S)-2-fluorenylmethoxycarbonylamino-2-cyclohexyl]acetyl]-4-fluoropyrrolidine (0.73 g).
MS(ESI pos.)m/z: 254([M+H]$^+$), 276([M+Na]$^+$), (ESI neg.)m/z: 252([M-H]-), 288([M+CL]$^-$).

Example 41

Synthesis of (2S,4S)-1-[(2S)-2-amino-4-[[5-(benzyloxycarbonyl)pentylamino]carbonyl]butanoyl]-2-cyano-4-fluoropyrrolidine hydrochloride

According to the manner similar to that of Example 40, the title compound (0.16 g) was obtained as a colorless amorphous substance from (2S,4S)-2-(aminocarbonyl)-4-fluoropyrrolidine hydrochloride (0.30 g) and [(2S)-2-fluorenylmethoxycarbonylamino-4-[(5-benzyloxycarbonylpentyl)aminocarbonyl]butanoic acid (1.07 g).
MS(ESI pos.)m/z: 447([M+H]$^+$), 469([M+Na]$^+$), (ESI neg.)m/z: 445([M-H]$^-$), 481([M+CL]$^-$).

Example 42

Synthesis of (2S,4S)-1-[[(2S)-2-amino-6-benzyloxycarbonylamino]hexanoyl]-2-cyano-4-fluoropyrrolidine hydrochloride

(1) Synthesis of (2S,4S)-2-(aminocarbonyl)-1-[[(2S)-2-(tert-butoxycarbonylamino)-6-benzyloxycarbonylamino] hexanoyl]-4-fluoropyrrolidine

In dimethylformamide (5 mL) were dissolved (2S,4S)-2-(aminocarbonyl)-4-fluoropyrrolidine hydrochloride (0.30 g) and hydroxysuccinimide (2S)-2-tert-butoxycarbonylamino-6-[benzyloxycarbonylaminolhexanoate (0.71 g), and then diisopropylamine (0.26 mL) was added, followed by stirring at room temperature overnight. Working-up in the manner similar to that of Example 1(3), the title compound (0.69 g) was obtained as a colorless amorphous substance.
MS(ESI pos.)m/z: 517([M+Na]$^+$), (ESI neg.)m/z: 493([M-H]$^-$).

(2) Synthesis of (2S,4S)-1-[[(2S)-2-(tert-butoxycarbonylamino)-6-benzyloxycarbonylamino]hexanoyl]-2-cyano-4-fluoropyrrolidine

According to the manner similar to that of Example 1(4), the title compound (0.36 g) was obtained as a colorless amorphous substance from (2S,4S)-2-(aminocarbonyl)-1-[[(2S)-2-(tert-butoxycarbonylamino)-6-benzyloxycarbonylamino]hexanoyl]-4-fluoropyrrolidine (0.65 g).
MS(ESI pos.)m/z: 499([M+Na]$^+$), (ESI neg.)m/z: 475([M-H]$^-$).

(3) Synthesis of (2S,4S)-1-[[(2S)-2-amino-6-benzyloxycarbonylamino]hexanoyl]-2-cyano-4-fluoropyrrolidine hydrochloride

In 1,4-dioxane (2.0 mL) was dissolved (2S,4S)-1-[[(2S)-2-(tert-butoxycarbonylamino)-6-benzyloxycarbonylamino]hexanoyl]-2-cyano-4-fluoropyrrolidine (0.34 g), and then 4M hydrochloric acid (1,4-dioxane solution, 2.0 mL) was added, followed by stirring at room temperature for 2.5 hours. The solution was concentrated under reduced pressure, and the resulting residue was dissolved by adding 2-propanol (3.0 mL) and methanol (1.0 mL), and then isopropyl ether (10 mL) was added. The solution was stirred at room temperature for 0.5 hour, the precipitated insoluble substance was collected by filtration to give the title compound (0.27 g) as a colorless powder.

MS(ESI pos.)m/z: 377($[M+H]^+$), 399($[M+Na]^+$), (ESI neg.)m/z: 375($[M-H]^-$), 411($[M+CL]^-$).

Example 43

Synthesis of (2S,4S)-2-cyano-4-fluoro-1-[[(2S,3S)-3-methyl-2-methylamino]pentanoyl]pyrrolidine hydrochloride

(1) Synthesis of (2S,4S)-2-(aminocarbonyl)-1-[(2S,3S)-2-[N-(tert-butoxycarbonyl)-N-methyl]amino-3-methylpentanoyl]-4-fluoropyrrolidine

According to the manner similar to that of Example 1(3), the title compound (1.35 g) was obtained as a colorless amorphous substance from (2S,4S)-2-(aminocarbonyl)-4-fluoropyrrolidine hydrochloride (0.67 g) and (2S,3S)-2-[N-(tert-butoxycarbonyl)-N-methyl]amino-3-methylpentanoic acid (0.98 g).

MS(ESI pos.)m/z: 382($[M+Na]^+$), (ESI neg.)m/z: 358($[M-H]^-$ ).

(2) Synthesis of (2S,4S)-1-[(2S,3S)-2-[N-(tert-butoxycarbonyl)-N-methyl]amino-3-methylpentanoyl]-2-cyano-4-fluoropyrrolidine

According to the manner similar to that of Example 15(2), the title compound (0.89 g) was obtained as a colorless amorphous substance from (2S,4S)-2-(aminocarbonyl)-1-[(2S,3S)-2-(N-(tert-butoxycarbonyl)-N-methyl]amino-3-methylpentanoyl]-4-fluoropyrrolidine (1.32 g).

MS(ESI pos.)m/z: 364($[M+Na]^+$), (ESI neg.)m/z: 340([M-H]-).

(3) Synthesis of (2S,4S)-2-cyano-4-fluoro-1-[[(2S,3S)-3-methyl-2-methylamino]pentanoyl]pyrrolidine hydrochloride

According to the manner similar to that of Example 15(3), the title compound (0.053 g) was obtained as a colorless powder from (2S,4S)-1-[(2S,3S)-2-[N-(tert-butoxycarbonyl)-N-methyl]amino-3-methylpentanoyl]-2-cyano-4-fluoropyrrolidine (0.10 g).

MS(ESI pos.)m/z: 242($[M+H]^+$), 264($[M+Na]^+$), (ESI neg.)m/z: 276($[M+CL]^-$).

Example 44

Synthesis of (2S,4S)-1-[[(2S,3R)-2-amino-3-(tert-butoxy)]butanoyl]-2-cyano-4-fluoropyrrolidine hydrochloride

(1) Synthesis of (2S,4S)-1-(tert-butoxycarbonyl)-2-cyano-4-fluoropyrrolidine

According to the manner similar to that of Example 15(2), the title compound (9.23 g) was obtained as a colorless powder from (2S,4S)-2-(aminocarbonyl)-1-(tert-butoxycarbonyl)-4-fluorppyrrolidine (10.0 g).

MS(ESI pos.)m/z: 237($[M+Na]^+$).

(2) Synthesis of (2S,4S)-2-cyano-4-fluoropyrrolidine hydrochloride

According to the manner similar to that of Example 15(3), the title compound (5.76 g) was obtained as a pale pink powder from (2S,4S)-1-(tert-butoxycarbonyl)-2-cyano-4-fluoropyrrolidine (8.99 g).

MS(ESI pos.)m/z: 115($[M+H]^+$), (EI pos.)m/z: 114($[M]^+$).

(3) Synthesis of (2S,4S)-2-cyano-1-[[(2S,3R)-2-fluorenylmethoxycarbonylamino-3-(tert-butoxy)]butanoyl]-4-fluoropyrrolidine

According to the manner similar to that of Example 1(3), the title compound (0.87 g) was obtained as a colorless

amorphous substance from (2S,4S)-2-cyano-4-fluoropyrrolidine hydrochloride (0.30 g) and [(2S,3R)-2-fluorenylmethoxycarbonylamino-3-(tert-butoxy)]butanoic acid (0.80 g).

MS(ESI pos.)m/z: 516([M+Na]$^+$).

(4) Synthesis of (2S,4S)-1-[[(2S,3R)-2-amino-3-(tert-butoxy)]butanoyl] -2-cyano-4-fluoropyrrolidine hydrochloride

According to the manner similar to that of Example 1(5), the title compound (0.28 g) was obtained as a colorless powder from (2S,4S)-2-cyano-1-[[(2S,3R)-2-fluorenylmethoxycarbonylamino-3-(tert-butoxy)]butanoyl]-4-fluoropyrrolidine (0.71 g).

MS(ESI pos.)m/z: 272([M+H]$^+$), 294([M+Na]$^+$), (ESI neg.)m/z: 306([M+CL]$^-$).

Example 45

Synthesis of (2S,4S)-2-cyano-4-fluoro-1-[[(3S)-1,2,3,4-tetrahydroisoquinolin-3-yl]carbonyl]pyrrolidine hydrochloride

(1) Synthesis of (2S,4S)-1-[(3S)-2-(tert-butoxycarbonyl)-1,2,3,4-tetrahydroisoquinolin-3-yl]carbonyl-2-cyano-4-fluoropyrrolidine

According to the manner similar to that of Example 1(3), the title compound (0.83 g) was obtained as a pale brown powder from (2S,4S)-2-cyano-4-fluoropyrrolidine hydrochloride (0.69 g) and (3S)-2-(tert-butoxycarbonyl)-1,2,3,4-tetrahydroisoquinoline-3-carboxylic acid (0.41 g).

MS(ESI pos.)m/z: 396([M+Na]$^+$), (ESI neg.)m/z: 372([M-H]$^-$).

(2) Synthesis of (2S,4S)-2-cyano-4-fluoro-1-[[(3S)-1,2,3,4-tetrahydroisoquinolin-3-yl]carbonyl]pyrrolidine hydrochloride

In ethanol (6 mL) was suspended (2S,4S)-1-[(3S)-2-(tert-butoxycarbonyl)-1,2,3,4-tetrahydroisoquinolin-3-yl] carbonyl-2-cyano-4-fluoropyrrolidine (0.30 g) and, after ice-cooling, 6M aqueous hydrochloric acid solution (3 mL) was added. The temperature was gradually raised to room temperature, and then, the mixture was stirred overnight. Further, methanol (6 mL) and 6M aqueous hydrochloric acid solution (3 mL) were added, followed by stirring at room temperature for a day. The solvent was concentrated under reduced pressure, and the resulting residue was washed with diethyl ether, dissolved in methanol (2 mL) and added dropwise to ethyl acetate (10 mL). To the solution was added diethyl ether (10 mL), and the precipitated insoluble substance was collected by filtration, and washed with ethyl acetate - diethyl ether (1:1) to give the title compound (0.19 g) as a pale brown powder.

MS(ESI pos.)m/z: 274([M+H]$^+$), 296([M+Na]$^+$), (ESI neg.)m/z: 272([M-H]$^-$), 308([M+CL]$^-$).

Example 46

Synthesis of (2S,4S)-2-cyano-4-fluoro-1-[[(3S)-7-hydroxy-1,2,3,4-tetrahydroisoquinolin-3-yl]carbonyl]pyrrolidine hydrochloride

(1) Synthesis of (2S,4S)-1-[(3S)-2-(tert-butoxycarbonyl)-7-hydroxy-1,2,3,4-tetrahydroisoquinolin-3-yl]carbonyl-2-cyano-4-fluoropyrrolidine

According to the manner similar to that of Example 1(3), the title compound (2.81 g) was obtained as a colorless amorphous substance from (2S,4S)-2-cyano-4-fluoropyrrolidine hydrochloride (1.54 g) and (3S)-2-(tert-butoxycarbonyl)-7-hydroxy-1,2,3,4-tetrahydroisoquinoline-3-carboxylic acid (3.00 g).

MS(ESI pos.)m/z: 412([M+Na]$^+$), (ESI neg.)m/z: 388([M-H]$^-$).

(2) Synthesis of (2S,4S)-2-cyano-4-fluoro-1-[[(3S)-7-hydroxy-1,2,3,4-tetrahydroisoquinolin-3-yl]carbonyl]pyrrolidine hydrochloride

According to the manner similar to that of Example 45(2), the title compound (0.12 g) was obtained as a pale brown powder from (2S,4S)-1-[(3S)-2-(tert-butoxycarbonyl)-7-hydroxy-1,2,3,4-tetrahydroisoquinolin-3-yl]carbonyl-2-cyano-4-fluoropyrrolidine (0.30 g).

MS(ESI pos.)m/z: 290([M+H]$^+$), 312([M+Na]$^+$), (ESI neg.)m/z: 288([M-H]$^-$), 324([M+CL]$^-$).

Example 47

Synthesis of (2S,4S)-2-cyano-4-fluoro-1-[[(3S)-7-methoxy-1,2,3,4-tetrahydroisoquinolin-3-yl]carbonyl]pyrrolidine hydrochloride

(1) Synthesis of (2S,4S)-1-[(3S)-2-(tert-butoxycarbonyl)-7-methoxy-1,2,3,4-tetrahydroisoquinolin-3-yl]carbonyl-2-cyano-4-fluoropyrrolidine

In N,N-dimethylformamide (5 mL) was dissolved (2S,4S)-1-[(3S)-2-(tert-butoxycarbonyl)-7-hydroxy-1,2,3,4-tetrahydroisoquinolin-3-yl]carbonyl-2-cyano-4-fluoropyrrolidine (0.63 g), and then methyl iodide (0.15 mL) and potassium carbonate (0.25 g) were added, followed by stirring at room temperature overnight. The reaction solution was taken up in water and extracted with ethyl acetate. The organic phase was washed with 0.5 M aqueous hydrochloric acid solution, a saturated aqueous sodium bicarbonate solution and a saturated aqueous sodium chloride solution, successively. After drying over anhydrous sodium sulfate, the drying agent was separated by filtration, and the filtrate was concentrated under reduced pressure. The residue was washed with diisopropyl ether to give the title compound (0.42 g) as a pale yellow powder.

MS(ESI pos.)m/z: 426($[M+Na]^+$), (ESI neg.)m/z: 402($[M-H]^-$).

(2) Synthesis of (2S,4S)-2-cyano-4-fluoro-1-[[(3S)-7-methoxy-1,2,3,4-tetrahydroisoquinolin-3-yl]carbonyl]pyrrolidine hydrochloride

According to the manner similar to that of Example 45(2), the title compound (0.12 g) was obtained as a pale brown powder from (2S,4S)-1-[(3S)-2-(tert-butoxycarbonyl)-7-methoxy-1,2,3,4-tetrahydroisoquinolin-3-yl]carbonyl-2-cyano-4-fluoropyrrolidine (0.30 g).

MS(ESI pos.)m/z: 304($[M+H]^+$), 326($[M+Na]^+$), (ESI neg.)m/z: 338($[M+CL]^-$).

Example 48

Synthesis of (2S,4S)-1-[(3S)-7-aminocarbonylmethoxy-1,2,3,4-tetrahydroisoquinolin-3-yl]carbonyl-2-cyano-4-fluoropyrrolidine hydrochloride

(1) Synthesis of (2S,4S)-1-[(3S)-2-(tert-butoxycarbonyl)-7-aminocarbonylmethoxy-1,2,3,4-tetrahydroisoquinolin-3-yl]carbonyl-2-cyano-4-fluoropyrrolidine

According to the manner similar to that of Example 47(1), the title compound (0.55 g) was obtained as a colorless powder from (25,4S)-1-[(3S)-2-(tert-butoxycarbonyl)-7-hydroxy-1,2,3,4-tetrahydroisoquinolin-3-yl]carbonyl-2-cyano-4-fluoropyrrolidine (0.60 g) and bromoacetamide (0.32 g).

MS(ESI pos.)m/z: 469($[M+Na]^+$), (ESI neg.)m/z: 445($[M-H]^-$).

(2) Synthesis of (2S,4S)-1-[(3S)-7-aminocarbonylmethoxy-1,2,3,4-tetrahydroisoquinolin-3-yl]carbonyl-2-cyano-4-fluoropyrrolidine hydrochloride

In ethyl acetate (12 mL) was suspended (2S,4S)-1-[(3S)-2-(tert-butoxycarbonyl)-7-aminocarbonylmethoxy-1,2,3,4-tetrahydroisoquinolin-3-yl]carbonyl-2-cyano-4-fluoropyrrolidine (0.30 g), and then 4M hydrochloric acid (ethyl acetate solution, 12 mL) was added with ice-cooling, followed by stirring with ice-cooling for 15 minutes and then at room temperature for an hour. Ethyl acetate (12 mL) was added, the resulting insoluble substance was collected by filtration and washed with ethyl acetate to give a pale yellow powder. The resulting powder was then suspended in ethanol (5 mL), stirred at room temperature for an hour, collected by filtration and washed with ethanol to give the title compound (0.21 g) as a colorless powder.

MS(ESI pos.)m/z: 347($[M+H]^+$), 369($[M+Na]^+$), (ESI neg.)m/z: 345([M-H]-), 381($[M+CL]^-$).

Example 49

Synthesis of (2S,4S)-2-cyano-1-[(3S)-6,7-dimethoxy-1,2,3,4-tetrahydroisoquinolin-3-yl]carbonyl-4-fluoropyrrolidine hydrochloride

(1) Synthesis of (3S)-2-(tert-butoxycarbonyl)-6,7-dimethoxy-1,2,3,4-tetrahydroisoquinoline-3-carboxylic acid

In tetrahydrofuran (6 mL) was suspended (3S)-6,7-dimethoxy-1,2,3,4-tetrahydroisoquinoline-3-carboxylate p-toluenesulfonate (0.50 g), and then a saturated aqueous sodium bicarbonate solution (3 mL) was added at room temperature, followed by stirring at room temperature until bubbles were no longer formed. The solution was cooled on an ice-bath, di-tert-butyldicarbonate (0.31 mL) was added, the temperature was gradually raised, and then, the mixture was stirred at room temperature overnight. 1M aqueous hydrochloric acid solution (5 mL) and an excess amount of sodium chloride were added, followed by extraction with ethyl acetate. The organic phase was washed with a saturated aqueous sodium chloride solution and, after drying over anhydrous sodium sulfate, the drying agent was removed by filtration, and the filtrate was concentrated under reduced pressure. The residue was washed with hexane - diisopropyl ether to give the title compound (0.38 g) as a pale yellow powder.

MS(ESI pos.)m/z: 360($[M+Na]^+$), (ESI neg.)m/z: 336([M-H]-).

(2) Synthesis of (2S,4S)-1-[(3S)-2-(tert-butoxycarbonyl)-6,7-dimethoxy-1,2,3,4-tetrahydroisoquinolin-3-yl]carbonyl-2-cyano-4-fluoropyrrolidine

According to the manner similar to that of Example 1(3), the title compound (0.34 g) was obtained as a colorless powder from (2S,4S)-2-cyano-4-fluoropyrrolidine hydrochloride (0.15 g) and (3S)-2-(tert-butoxycarbonyl)-6,7-dimethoxy-1,2,3,4-tetrahydroisoquinoline-3-carboxylic acid (0.34 g).

MS(ESI pos.)m/z: 456($[M+Na]^+$).

(3) Synthesis of (2S,4S)-2-cyano-1-[(3S)-6,7-dimethoxy-1,2,3,4-tetrahydroisoquinolin-3-yl]carbonyl-4-fluoropyrrolidine hydrochloride

According to the manner similar to that of Example 45(2), the title compound (0.20 g) was obtained as a pale yellow powder from (2S,4S)-1-[(3S)-2-(tert-butoxycarbonyl)-6,7-dimethoxy-1,2,3,4-tetrahydroisoquinolin-3-yl]carbonyl-2-cyano-4-fluoropyrrolidine (0.30 g).

MS(ESI pos.)m/z: 334$[M+H]^+$), 356($[M+Na]^+$), (ESI neg.)m/z: 368($[M+CL]^-$).

Example 50

Synthesis of (2S,4S)-1-[[(2S,3R)-2-amino-3-benzyloxy]butanoyl]-2-cyano-4-fluoropyrrolidine hydrochloride

(1) Synthesis of (2S,4S)-1-[[(2S,3R)-2-(tert-butoxycarbonylamino)-3-benzyloxy]butanoyl]-2-cyano-4-fluoropyrrolidine

According to the manner similar to that of Example 1(3), the title compound (0.81 g) was obtained as a pale brown amorphous substance from (2S,4S)-2-cyano-4-fluoropyrrolidine hydrochloride (0.30 g) and [(2S,3R)-2-(tert-butoxycarbonylamino)-3-benzyloxy]butanoic acid (0.62 g).

MS(ESI pos.)m/z: 406($[M+H]^+$), 428($[M+Na]^+$), (ESI neg.)m/z: 404($[M-H]^-$).

(2) Synthesis of (2S,4S)-1-[[(2S,3R)-2-amino-3-benzyloxy]butanoyl]-2-cyano-4-fluoropyrrolidine hydrochloride

According to the manner similar to that of Example 48(2), the title compound (0.34 g) was obtained as a brown powder from (2S,4S)-1-[[(2S,3R)-2-(tert-butoxycarbonylamino)-3-benzyloxy]butanoyl]-2-cyano-4-fluoropyrrolidine (0.49 g).

MS(ESI pos.)m/z: 306($[M+H]^+$), 328($[M+Na]^+$), (ESI neg.)m/z: 340($[M+CL]^-$).

Example 51

Synthesis of (2S,4S)-2-cyano-4-fluoro-1-[[(2S)-pyrrolidin-2-yl]carbonyl]pyrrolidine hydrochloride

(1) Synthesis of (2S,4S)-2-(aminocarbonyl)-1-[(2S)-1-(tert-butoxycarbonyl)pyrrolidin-2-yl]carbonyl-4-fluoropyrrolidine

According to the manner similar to that of Example 1(3), the title compound (0.88 g) was obtained as a colorless amorphous substance from (2S,4S)-2-aminocarbonyl-4-fluoropyrrolidine hydrochloride (0.50 g) and (2S)-1-(tert-butoxycarbonyl)-2-pyrrolidinecarboxylic acid (0.64 g).
MS(ESI pos.)m/z: 352($[M+Na]^+$), (ESI neg.)m/z: 328($[M-H]^-$).

(2) Synthesis of (2S,4S)-1-[(25)-1-(tert-butoxycarbonyl)pyrrolidin-2-yl]carbonyl-2-cyano-4-fluoropyrrolidine

According to the manner similar to that of Example 11(3), the title compound (0.65 g) was obtained as a colorless solid from (2S,4S)-2-(aminocarbonyl)-1-[(2S)-1-(tert-butoxycarbonyl)pyrrolidin-2-yl]carbonyl-4-fluoropyrrolidine (0.80 g).
MS(ESI pos.)m/z: 334($[M+Na]^+$).

(3) Synthesis of (2S,4S)-2-cyano-4-fluoro-1-[[(2S)pyrrolidin-2-yl]carbonyl]pyrrolidine hydrochloride

In diethyl ether (10 mL) was suspended (2S,4S)-1-[(2S)-1-(tert-butoxycarbonyl)pyrrolidin-2-yl]carbonyl-2-cyano-4-fluoropyrrolidine (0.66 g), and then 4M hydrochloric acid (dioxane solution, 15 mL) was added, followed by stirring at room temperature for 3.5 hours. After evaporation of the solvent, diisopropyl ether (15 mL) was added to the residue, followed by stirring. The precipitated insoluble substance was collected by filtration to give the title compound (0.53 g) as a pale orange solid.
MS(ESI pos.)m/z: 212($[M+H]^+$), 234($[M+Na]^+$), (ESI neg.)m/z: 246($[M+CL]^-$).

Example 52

Synthesis of (2S,4S)-1-[(cis-2-amino-cyclopentan-1-yl)carbonyl]-2-cyano-4-fluoropyrrolidine hydrochloride

(1) Synthesis of (2S,4S)-1-[(cis-2-(tert-butoxycarbonylamino)-cyclopentan-1-yl)carbonyl]-2-cyano-4-fluoropyrrolidine

The crude product which was obtained from (2S,4S)-2-cyano-4-fluoropyrrolidine hydrochloride (0.30 g) and cis-2-(tert-butoxycarbonylamino)-cyclopentane-1-carboxylic acid (0.50 g) according to the manner similar to that of Example 1(3) was separated by a silica gel column chromatography (developing solvent; hexane : ethyl acetate =4:1 - 3:2) to give two diastereoisomers of the title compound, a lower polar component (0.25 g) as a colorless powder and a higher polar component (0.27 g) as a colorless powder.
Lower polar component; Silica gel TLC, Rf:0.17 (developing solvent; hexane : ethyl acetate =1:1), MS(ESI pos.)m/z: 348($[M+Na]^+$), (ESI neg.)m/z: 324($[M-H]^-$).
Higher polar component; Silica gel TLC, Rf:0.10 (developing solvent; hexane : ethyl acetate =1:1), MS(ESI pos.)m/z: 348($[M+Na]^+$), (ESI neg.)m/z: 324($[M-H]^-$).

(2) Synthesis of (2S,4S)-1-[(cis-2-aminocyclopentan-1-yl)carbonyl]-2-cyano-4-fluoropyrrolidine hydrochloride

According to the manner similar to that of Example 48(2), the title compound (0.060 g) was obtained as a colorless powder from lower polar ((2S,4S)-1-[(cis-2-(tert-butoxycarbonylamino)-cyclopentan-1-yl)carbonyl]-2-cyano-4-fluoropyrrolidine (0.10 g) obtained in Example 52(1).
MS(ESI pos.)m/z: 226($[M+H]^+$), 248($[M+Na]^+$), (ESI neg.)m/z: 260($[M+CL]^-$).

Example 53

Synthesis of (2S,4S)-1-[(cis-2-amino-cyclopentan-1-yl)carbonyl]-2-cyano-4-fluoropyrrolidine hydrochloride

According to the manner similar to that of Example 48(2), the title compound (0.067 g) was obtained as a colorless powder from higher polar ((2S,4S)-1-[(cis-2-tert-butoxycarbonylamino)-cyclopentan-1-yl)carbonyl]-2-cyano-4-fluoropyrrolidine (0.10 g) obtained in Example 52(1).
MS(ESI pos.)m/z: 226($[M+H]^+$), 248($[M+Na]^+$), (ESI neg.)m/z: 260($[M+CL]^-$).

Example 54

Synthesis of (2S,4S)-2-(aminocarbonyl)-1-(tert-butoxycarbonyl)-4-fluoropyrrolidine

In dichloromethane (20 mL) was suspended (2S,4R)-2-(aminocarbonyl)-1-(tert-butoxycarbonyl)-4-hydroxypyrrolidine (2.0 g) obtained in Reference Example 3, and diethylaminosulfur trifluoride (2.3 mL) was added dropwise with cooling (-78°C) on a dry ice - acetone bath. The temperature was gradually raised to room temperature, and then, the mixture was stirred for 6 hours. The reaction mixture was taken up in a saturated aqueous sodium bicarbonate solution and extracted with ethyl acetate. The organic phase was washed with a saturated aqueous sodium bicarbonate solution and a saturated aqueous sodium chloride solution, successively, and dried over anhydrous sodium sulfate. After removal of the drying agent by filtration, the filtrate was concentrated under reduced pressure. The residue was purified by a silica gel column chromatography (developing solvent; hexane : ethyl acetate =4:1 - 1:5) to give the title compound (300 mg) as a colorless solid.

MS(ESI pos.)m/z: 255[[M+Na]$^+$), (ESI neg.)m/z: 231([M-H]$^-$)

Results of a high resolution mass spectrography of the compounds in the above-mentioned examples are shown in the following table.

| Example Number | Ionization method | Detected ion | Compositional formula | Calcd. value | Found value |
|---|---|---|---|---|---|
| 1 | ESI | [M+H]$^+$ | $C_{11} H_{19} F N_3 O$ | 228.1512 | 228.1508 |
| 3 | ESI | [M+H]$^+$ | $C_{11} H_{19} F N_3 O$ | 228.1512 | 228.1518 |
| 4 | ESI | [M+H]$^+$ | $C_{14} H_{18} F N_6 O_3$ | 337.1424 | 337.1440 |
| 5 | ESI | [M+H]$^+$ | $C_{15} H_{18} F N_6 O$ | 317.1526 | 317.1536 |
| 6 | ESI | [M+H]$^+$ | $C_{12} H_{22} N_3 O_2$ | 240.1712 | 240.1723 |
| 7 | ESI | [M+H]$^+$ | $C_{12} H_{22} N_3 O_2$ | 241.1712 | 240.1712 |
| 8 | ESI | [M+H]$^+$ | $C_{11} H_{20} N_3 O_2$ | 226.1556 | 226.1570 |
| 9 | ESI | [M+H]$^+$ | $C_{11} H_{20} N_3 O_2$ | 226.1556 | 226.1565 |
| 10 | ESI | [M+H]$^+$ | $C_{11} H_{19} Cl N_3 O$ | 244.1217 | 244.1231 |
| 11 | ESI | [M+H]$^+$ | $C_{11} H_{18} N_3 O_2$ | 224.1399 | 224.1381 |
| 12 | ESI | [M+H]$^+$ | $C_{11} H_{20} N_3 O_2$ | 226.1556 | 226.1545 |
| 13 | ESI | [M+H]$^+$ | $C_{11} H_{19} F N_3 O$ | 228.1512 | 228.1525 |
| 14 | EI | [M]$^+$ | $C_{11} H_{17} F_2 N_3 O$ | 245.1340 | 245.1353 |
| 15 | EI | [M]$^+$ | $C_{10} H_{16} F N_3 O$ | 213.1277 | 213.1284 |
| 16 | EI | [M]$^+$ | $C_{10} H_{16} F N_3 O_2$ | 229.1227 | 229.1232 |
| 17 | EI | [M]$^+$ | $C_{11} H_{18} F N_3 O$ | 227.1434 | 227.1455 |
| 19 | EI | [M]$^+$ | $C_{11} H_{18} F N_3 O$ | 227.1434 | 227.1438 |
| 21 | ESI | [M+H]$^+$ | $C_{11} H_{19} F N_3 O$ | 228.1512 | 228.1521 |
| 22 | EI | [M]$^+$ | $C_{10} H_{16} F N_3 O$ | 213.1277 | 213.1297 |
| 23 | ESI | [M+H]$^+$ | $C_{10} H_{15} F N_3 O$ | 212.1199 | 212.1208 |
| 24 | ESI | [M+H]$^+$ | $C_{11} H_{17} F N_3 O$ | 226.1356 | 226.1352 |
| 25 | ESI | [M+H]$^+$ | $C_{12} H_{19} F N_3 O$ | 240.1512 | 240.1499 |
| 26 | ESI | [M+H]$^+$ | $C_{13} H_{21} F N_3 O_2$ | 270.1618 | 270.1621 |
| 27 | ESI | [M+H]$^+$ | $C_{13} H_{23} F N_3 O_2$ | 272.1774 | 272.1785 |
| 28 | EI | [M]$^+$ | $C_{15} H_{24} F N_3 O$ | 281.1903 | 281.1911 |
| 29 | EI | [M]$^+$ | $C_{17} H_{22} F N_3 O_3$ | 335.1645 | 335.1670 |
| 30 | ESI | [M+H]$^+$ | $C_{12} H_{21} F N_3 O_2$ | 258.1618 | 258.1603 |

(continued)

| Example Number | Ionization method | Detected ion | Compositional formula | Calcd. value | Found value |
|---|---|---|---|---|---|
| 31 | ESI | $[M+H]^+$ | $C_{11} H_{19} F N_3 O_2$ | 244.1461 | 244.1449 |
| 32 | EI | $[M]^+$ | $C_{17} H_{24} F N_3 O$ | 305.1903 | 305.1913 |
| 33 | ESI | $[M+H]^+$ | $C_{17} H_{25} F N_3 O_2$ | 322.1931 | 322.1913 |
| 34 | ESI | $[M+H]^+$ | $C_{17} H_{25} F N_3 O_2$ | 322.1931 | 322.1949 |
| 35 | ESI | $[M+H]^+$ | $C_{18} H_{27} F N_3 O_2$ | 336.2087 | 336.2101 |
| 36 | ESI | $[M+H]^+$ | $C_{17} H_{25} F N_3 O$ | 306.1982 | 306.1973 |
| 37 | EI | $[M]^+$ | $C_{14} H_{17} Cl F N_5 O$ | 325.1106 | 325.1112 |
| 38 | EI | $[M]^+$ | $C_{14} H_{18} F N_5 O$ | 291.1495 | 291.1503 |
| 39 | ESI | $[M+H]^+$ | $C_{15} H_{20} F N_6 O_2$ | 335.1632 | 335.1638 |
| 40 | EI | $[M]^+$ | $C_{13} H_{20} F N_3 O$ | 253.1590 | 253.1605 |
| 41 | EI | $[M]^+$ | $C_{23} H_{31} F N_4 O_4$ | 446.2329 | 446.2328 |
| 42 | EI | $[M]^+$ | $C_{19} H_{25} F N_4 O_3$ | 376.1911 | 376.1930 |
| 43 | ESI | $[M+H]^+$ | $C_{12} H_{21} F N_3 O$ | 242.1669 | 242.1686 |
| 44 | ESI | $[M+H]^+$ | $C_{13} H_{23} F N_3 O_2$ | 272.1774 | 272.1793 |
| 45 | ESI | $[M+H]^+$ | $C_{15} H_{17} F N_3 O$ | 274.1356 | 274.1339 |
| 46 | ESI | $[M+H]^+$ | $C_{15} H_{17} F N_3 O_2$ | 290.1305 | 290.1304 |
| 47 | ESI | $[M+H]^+$ | $C_{16} H_{19} F N_3 O_2$ | 304.1461 | 304.1456 |
| 48 | ESI | $[M+H]^+$ | $C_{17} H_{20} F N_4O_3$ | 347.1519 | 347.1527 |
| 49 | ESI | $[M+H]^+$ | $C_{17} H_{21} F N_3 O_3$ | 334.1567 | 334.1566 |
| 50 | ESI | $[M+H]^+$ | $C_{16} H_{21} F N_3 O_2$ | 306.1618 | 306.1615 |
| 51 | EI | $[M]^+$ | $C_{10} H_{14} F N_3 O$ | 211.1121 | 211.1129 |
| 52 | ESI | $[M+H]^+$ | $C_{11} H_{17} F N_3 O$ | 226.1356 | 226.1355 |
| 53 | ESI | $[M+H]^+$ | $C_{11} H_{17} F N_3 O$ | 226.1356 | 226.1359 |

Experiment 1 [Dipeptidyl Peptidase IV Activity Inhibition Test]

An activity inhibition test of dipeptidyl peptidase IV (DPP IV) was carried out according to the method described in Diabetes, 47, 764 - 769 (1998). Plasma including dipeptidyl peptidase IV was prepared by centrifugation of blood collected from healthy human volunteers. Enzyme reaction was carried out using a plate with 96 flat bottom wells in the buffer solution containing 25 mM HEPES, 140 mM NaCl and 1% BSA, pH 7.8. To a mixture of 25 µl of 100 µM Gly-Pro-4-methylcoumaryl-7-amide solution (manufactured by Peptide Institute, Inc.), 7.5 µl of 133 mM magnesium chloride solution and 5 µl of the test compound was added 12.5 µl of plasma which was diluted to 1/100 with the above buffer solution. After the reaction at room temperature for 2 hours, 50 µl of 25% aqueous acetic acid solution was added to stop the reaction. The fluorescence intensity of the liberated 7-amino-4-methylcoumarine was determined by using a fluorescence plate reader (1420 ARVOTM Multilabel Counter; manufactured by Wallac Oy, Excitation: 390 nm, Emission: 460 nm). The fluorescence intensity at which the reaction time of the vehicle addition was 0 minute was regarded as a blank value, and the specific fluorescence intensity was calculated by subtracting the blank value from the determined value. The inhibition rate (%) of the dipeptidyl peptidase IV activity was calculated from the resulting specific fluorescence intensity according to the following formula. The dimethyl sulfoxide solution containing the test compound at high concentration of 1000-fold was prepared and diluted with the above-mentioned buffer solution for use. The concentration ($IC_{50}$) of the test compound to exhibit 50% inhibition was calculated from the inhibition rate at each concentration.

Inhibition rate (%) = A/Bx100

(A= fluorescence intensity of vehicle addition-fluorescence intensity of the test compound addition)
(B = fluorescence intensity of the vehicle addition)

As the comparative drugs were used 1-[(2S,3S)-2-amino-3-methylpentanoyl]-2-(S)-cyanopyrrolidine trifluoroacetate (Compound A) described in Patent WO95/15309 and 1-[2-[(5-cyanopyridin-2-yl)amino]ethylamino]acetyl-2-(S)-cyanopyrrolidine dihydrochloride (Compound B) described Patent WO98/19998 or US. Patent 6011155.

Results are shown in Table 1. The compounds of the present invention are confirmed to have an enhancing activity by introducing a fluorine atom on the pyrrolidine ring, and confirmed to have an excellent DPP IV inhibition activity.

Table 1

| DPP IV inhibition activity ($IC_{50}$ value, nM) | |
|---|---|
| Compound A | 1.5 |
| Compound of Example 1 | 0.6 |
| Compound of Example 15 | 0.7 |
| Compound of Example 17 | 0.6 |
| Compound B | 5.5 |
| Compound of Example 5 | 1.1 |
| Compound of Example 21 | 2.9 |
| Compound of Example 26 | 3.3 |

Experiment 2 [Concentration of test drug in blood by oral administration in rats]

Wistar rats, male (8 weeks old) were used after fasting from the day before the test. Aqueous solution of the compound of Example 1 or Compound A (each of which was prepared by dissolving in purified water to make up to 1 mg/ml aqueous solution) was orally administered at a dose of 1 mg/kg(1 ml/kg).

At 5, 10, 15, 30 minutes, 1 and 2 hours after the administration, each 0.2 ml of blood was collected from the jugular vein and, after centrifugation, the resulting plasma was used as sample.

Concentrations of the compound of Example 1 and Compound A in plasma were measured by liquid chromatography tandem mass spectrography method (LC/MS/MS). That is, 50 μl of the plasma was added to 200 μl of acetonitrile, stirred and, after centrifugation, the supernatant was injected to a liquid chromatography wherein CAPCEL PAK C18, UG120 5 μm (150 mm long, 2 mm diameter) was used as a column, a mixture of 10 mM aqueous ammonium acetate solution and 90% aqueous acetonitrile solution (1:9) was used as an eluent, Sciex API3000 LC/MS/MS System (Perkin Elmer Sciex) was used as a MS/MS condition, ESI was used as an ionization method, and cation, and SRM (precursor ion and daughter ion) was used for monitoring. Indicators of the compound of Example 1 were m/z 228.0 and m/z 86.0, and those of Compound A were m/z 210.1 and m/z 86.0.

Table 2 shows mean concentrations in plasma at the time when blood was collected after oral administration.

The compound of Example 1 has a higher concentration in plasma than Compound A, and is confirmed to increase the concentration in plasma by introducing a fluorine atom on the pyrrolidine ring.

Table 2

| Plasma concentrations of the drug after administration | | | | | | |
|---|---|---|---|---|---|---|
| | Concentration in plasma of drug : unit (ng/ml) | | | | | |
| | 5 min. | 10 min. | 15 min. | 30 min. | 1 hour | 2 hours |
| Compound of Example 1 | 157 | 372 | 348 | 195 | 24 | 5 |
| Compound A | 122 | 146 | 113 | 28 | 6 | 2 |
| (Each value is a mean value of 2 samples) | | | | | | |

Experiment 3 [Effect of dipeptidyl peptidase IV inhibitors on oral glucose tolerance test (OGTT) in Zucker fatty rats]

An OGTT in Zucker fatty rats was carried out referring to the method described in Diabetologia, 42, 1324 -

1331 (1999). In the test, male Zucker fatty rats, 10 weeks old (Charles River Japan, Inc.) were used after fasting for 16 hours. Rats had free access to water up to the time just before the test, and did not have up to the end of the test. Before the begining of the test, blood was collected from the intraorbital vein using a heparin-treated tube for blood collection (manufactured by Drummond Scientific Co.). The test compounds were each dissolved in water for injection under Japanese Pharmacopoeia (manufactured by Hikari Pharmaceutical Co.) and orally administered in an amount of 2 ml/kg of the body weight. The same amount of water for injection under Japanese Pharmacopoeia only was administered to control group. One g/kg of the body weight of glucose was dissolved in water for injection under Japanese Pharmacopoeia and orally administered in an amount of 2 ml/kg of the body weight at 30 minutes after the administration of the test compound or water for injection. Blood was collected from the ophthalmic vein at 15, 30 and 60 minutes after the glucose administration. Blood samples were immediately mixed with heparin (manufactured by Shimizu Pharmaceutical Co., Ltd.), and centrifuged at 3000 rpm for 15 minutes at 4°C to recover plasmas, which were immediately frozen.

Plasma glucose levels of the frozen samples (mg/dl) were determined using Glucose CII test Wako (manufactured by Wako Fine Chemical Industry Co.), and the area under the curve (min·mg/dl) was calculated from the plasma glucose levels which were determined from the blood collected for 60 minutes after the glucose administration. On the other hand, the plasma glucose level of the sample which was obtained from the blood collected before the beginning of the test was used as substitutes for the plasma glucose level at 0 minute.

Results are shown in Table 3. The compound of Example 1 inhibited significantly the elevation of the plasma glucose level ($p<0.05$).

Table 3

| | mean ± S.E. (min·mg/dl) |
|---|---|
| Water-administered group | 15545 ± 765 |
| Compound of Example 1-administered group | 13248 ± 619 |

INDUSTRIAL APPLICABILITY

The present invention makes it possible to provide compounds having an excellent dipeptidyl peptidase IV (DPP IV) inhibition activity, and the compounds of the present invention are useful as an agent for preventing or treating diabetes mellitus, immune diseases, etc.

## Claims

**1.** A cyanopyrrolidine derivative represented by Formula (1):

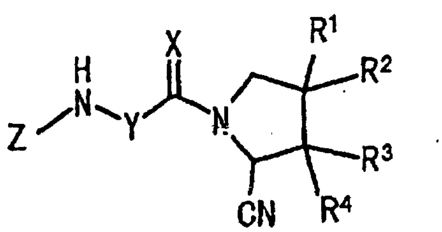

(1)

[wherein $R^1$ is a halogen atom, a hydroxyl group, an alkoxy group having 1 to 5 carbon atoms or an alkyl group having 1 to 5 carbon atoms, $R^2$ is a hydrogen atom, a halogen atom, a hydroxyl group, an alkoxy group having 1 to 5 carbon atoms or an alkyl group having 1 to 5 carbon atoms, or $R^1$ and $R^2$ together form an oxo, a hydroxyimino group, an alkoxyimino group having 1 to 5 carbon atoms or an alkylidene group having 1 to 5 carbon atoms,

$R^3$ and $R^4$ are each a hydrogen atom, a halogen atom, a hydroxyl group, an alkoxy group having 1 to 5 carbon atoms or an alkyl group having 1 to 5 carbon atoms, or $R^3$ and $R^4$ together form an oxo, a hydroxyimino group, an alkoxyimino group having 1 to 5 carbon atoms or an alkylidene group having 1 to 5 carbon atoms,

X is an oxygen atom or a sulfur atom,

Y is $-CR^5R^6-$ [wherein $R^5$ and $R^6$ are the same or different, and each a hydrogen atom; a halogen atom; an alkyl group having 1 to 10 carbon atoms which is optionally substituted with at least one selected from the group

consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), $-CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a chain alkyl group having 1 to 5 carbon atoms or a benzyl group); or an alkenyl group having 2 to 10 carbon atoms which is optionally substituted with at least one selected from the group consisting of a halogen atom, a hydroxyl group, a carboxyl group, an amino group, an aminocarbonyl group and a chain alkoxy group having 1 to 5 carbon atoms],

or $-CR^7R^8-CR^9R^{10}-$ (wherein $R^7$, $R^8$, $R^9$ and $R^{10}$ are the same or different, and each a hydrogen atom; a halogen atom; or an alkyl group having 1 to 10 carbon atoms which is optionally substituted with at least one selected from the group consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), $-CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a chain alkyl group having 1 to 5 carbon atoms or a benzyl group), or $R^7$ and $R^9$ together with the carbon atom to which they are attached form a cycloalkyl group having 3 to 8 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms; a cycloalkenyl group having 4 to 8 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms; a bicycloalkyl group having 5 to 10 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms; or a bicycloalkenyl group having 5 to 10 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms), and

Z is a hydrogen atom; or an alkyl group having 1 to 10 carbon atoms which is optionally substituted with at least one selected from the group consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), $-CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a chain alkyl group having 1 to 5 carbon atoms or a benzyl group),

or Y and Z together with the nitrogen atom to which they are attached form a cyclic amino group having 2 to 10 carbon atoms which is optionally substituted with at least one selected from the group consisting of a halogen atom, a hydroxyl group, an amino group, a chain alkyl group having 1 to 5 carbon atoms and $-OR^{15}$ (wherein $R^{15}$ is a chain alkyl group having 1 to 5 carbon atoms, an aminocarbonylmethyl group or a benzyl group)] or a pharmaceutically acceptable salt thereof.

**2.** The cyanopyrrolidine derivative of Formula (1) according to Claim 1 wherein $R^1$ is a halogen atom, a hydroxyl group, an alkoxy group having 1 to 5 carbon atoms or an alkyl group having 1 to 5 carbon atoms, and $R^2$, $R^3$ and $R^4$ are each a hydrogen atom, a halogen atom, a hydroxyl group, an alkoxy group having 1 to 5 carbon atoms or an alkyl group having 1 to 5 carbon atoms, or the pharmaceutically acceptable salt thereof.

**3.** The cyanopyrrolidine derivative of Formula (1) according to Claim 1 or 2 wherein $R^1$ is a fluorine atom or a chlorine atom, or the pharmaceutically acceptable salt thereof.

**4.** The cyanopyrrolidine derivative of Formula (1) according to Claim 1 or 2 wherein $R^1$ is a fluorine atom, and $R^2$ is a hydrogen atom, or the pharmaceutically acceptable salt thereof.

**5.** The cyanopyrrolidine derivative of Formula (1) according to Claim 1 or 2 wherein $R^1$ is a fluorine atom, and $R^2$, $R^3$ and $R^4$ are each a hydrogen atom, or the pharmaceutically acceptable salt thereof.

**6.** A cyanopyrrolidine derivative represented by Formula (2):

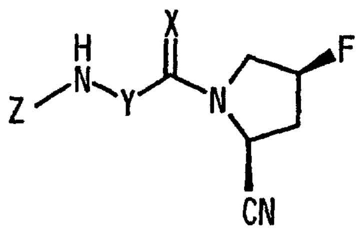

(2)

[wherein X is an oxygen atom or a sulfur atom,

Y is $-CR^5R^6-$ [wherein $R^5$ and $R^6$ are the same or different, and each a hydrogen atom; a halogen atom; an alkyl group having 1 to 10 carbon atoms which is optionally substituted with at least one selected from the group consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), - $CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a chain alkyl group having 1 to 5 carbon atoms or a benzyl group); or an alkenyl group having 2 to 10 carbon atoms which is optionally substituted with at least one selected from the group consisting of a halogen atom, a hydroxyl group, a carboxyl group, an amino group, an aminocarbonyl group and a chain alkoxy group having 1 to 5 carbon atoms],

or $-CR^7R^8-CR^9R^{10}-$ (wherein $R^7$, $R^8$, $R^9$ and $R^{10}$ are the same or different, and each a hydrogen atom; a halogen atom; or an alkyl group having 1 to 10 carbon atoms which is optionally substituted with at least one selected from the group consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), $-CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a chain alkyl group having 1 to 5 carbon atoms or a benzyl group), or $R^7$ and $R^9$ together with the carbon atom to which they are attached form a cycloalkyl group having 3 to 8 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms; a cycloalkenyl group having 4 to 8 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms; a bicycloalkyl group having 5 to 10 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms; or a bicycloalkenyl group having 5 to 10 carbon atoms which is optionally substituted with at least one selected from the group consisting of a halogen atom, a hydroxyl group, a carboxyl group, an amino group, an aminocarbonyl group, a chain alkyl group having 1 to 5 carbon atoms and a chain alkoxy group having 1 to 5 carbon atoms), and

Z is a hydrogen atom; or an alkyl group having 1 to 10 carbon atoms which is optionally substituted with at least one selected from the group consisting of a halogen atom, a hydroxyl group, a hydroxyalkyl group having 1 to 5 carbon atoms, a carboxyl group, a mercapto group, an alkylthio group having 1 to 5 carbon atoms, a guanidyl group, an optionally substituted phenyl group, an imidazolyl group, an indolyl group, $-NHR^{11}$ (wherein $R^{11}$ is a hydrogen atom, an optionally substituted phenyl group, an optionally substituted pyridyl group, a tert-butoxycarbonyl group or a benzyloxycarbonyl group), $-CONHR^{12}$ {wherein $R^{12}$ is a hydrogen atom or $-(CH_2)_m-R^{13}$ (wherein m is an integer of 1 to 5, and $R^{13}$ is a hydrogen atom, a methoxycarbonyl group, an ethoxycarbonyl group or a benzyloxycarbonyl group)} and $-OR^{14}$ (wherein $R^{14}$ is a chain alkyl group having 1 to 5 carbon atoms or a benzyl

group),
or Y and Z together with the nitrogen atom to which they are attached form a cyclic amino group having 2 to 10 carbon atoms which is optionally substituted with at least one selected from the group consisting of a halogen atom, a hydroxyl group, an amino group, a chain alkyl group having 1 to 5 carbon atoms and $-OR^{15}$ (wherein $R^{15}$ is a chain alkyl group having 1 to 5 carbon atoms, an aminocarbonylmethyl group or a benzyl group)] or a pharmaceutically acceptable salt thereof.

**7.** The cyanopyrrolidine derivative of Formula (1) or (2) according to any one of Claims 1 to 6 wherein X is an oxygen atom, or the pharmaceutically acceptable salt thereof.

**8.** The cyanopyrrolidine derivative of Formula (1) or (2) according to Claim 7 wherein Y is $-CH_2-$, or the pharmaceutically acceptable salt thereof.

**9.** The cyanopyrrolidine derivative of Formula (1) or (2) according to Claim 8 wherein Z is a branched or cyclic alkyl group having 4 to 10 carbon atoms which is optionally substituted with at least one selected from the group consisting of a hydroxyl group and a hydroxyalkyl group having 1 to 5 carbon atoms, or the pharmaceutically acceptable salt thereof.

**10.** The cyanopyrrolidine derivative of Formula (1) or (2) according to Claim 8 wherein Z is a tert-butyl group, a (1-hydroxymethyl)cyclopentyl group or a (2-hydroxy-1,1-dimethyl)ethyl group, or the pharmaceutically acceptable salt thereof.

**11.** The cyanopyrrolidine derivative of Formula (1) or (2) according to Claim 7 wherein Y is $-CR^5R^6-$ (wherein $R^5$ is a hydrogen atom) and Z is a hydrogen atom, or the pharmaceutically acceptable salt thereof.

**12.** The cyanopyrrolidine derivative of Formula (1) or (2) according to Claim 7 wherein Y is $-CR^5R^6-$ (wherein $R^5$ is a hydrogen atom, $R^6$ is a branched or cyclic alkyl group having 3 to 6 carbon atoms) and Z is a hydrogen atom, or the pharmaceutically acceptable salt thereof.

**13.** The cyanopyrrolidine derivative of Formula (1) or (2) according to Claim 7 wherein Y is $-CH[CH(CH_3)_2]-$, $-CH[C(CH_3)_3]-$ or $-CH[CH(CH_3)CH_2CH_3]-$ and Z is a hydrogen atom, or the pharmaceutically acceptable salt thereof.

**14.** A pharmaceutical preparation which comprises as an effective ingredient the cyanopyrrolidine derivative or the pharmaceutically acceptable salt thereof according to any one of Claims 1 to 13.

**15.** The pharmaceutical preparation according to Claim 14 for preventing or treating a disease or condition capable of being improved by inhibition of dipeptidyl peptidase IV.

**16.** The pharmaceutical preparation according to Claim 15 wherein the disease or condition capable of being improved by inhibition of dipeptidyl peptidase IV is diabetes mellitus.

**17.** The pharmaceutical preparation according to Claim 15 wherein the disease or condition capable of being improved by inhibition of dipeptidyl peptidase IV is an immune disease.

## INTERNATIONAL SEARCH REPORT

International application No.
PCT/JP01/09818

A. CLASSIFICATION OF SUBJECT MATTER
Int.Cl$^7$ C07D207/16, 401/12, 401/06, A61K31/401, 31/4439, 31/4725, A61P43/00, 3/10, 37/02, 29/00, 31/18, 35/04

According to International Patent Classification (IPC) or to both national classification and IPC

B. FIELDS SEARCHED

Minimum documentation searched (classification system followed by classification symbols)
Int.Cl$^7$ C07D207/16, 401/12, 401/06, A61K31/401, 31/4439, 31/4725

Documentation searched other than minimum documentation to the extent that such documents are included in the fields searched

Electronic data base consulted during the international search (name of data base and, where practicable, search terms used)
CA (STN), REGISTRY (STN), WPIDS (STN)

C. DOCUMENTS CONSIDERED TO BE RELEVANT

| Category* | Citation of document, with indication, where appropriate, of the relevant passages | Relevant to claim No. |
|---|---|---|
| A | JP 2000-511559 A (Novartis AG), 05 September, 2000 (05.09.2000), the whole document & WO 98/19998 A | 1-17 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 February, 2002 (01.02.02) | 12 February, 2002 (12.02.02) |
| Name and mailing address of the ISA/ Japanese Patent Office | Authorized officer |
| Facsimile No. | Telephone No. |

Form PCT/ISA/210 (second sheet) (July 1992)